# EUROPEAN PATENT APPLICATION

(11) **EP 4 538 352 A1**
(43) Date of publication of application: **16.04.2025**
(21) Application number: 23845518.2
(22) Date of filing: 25.07.2023
(51) Int. Cl.: C10G 55/04

(54) **METHOD AND SYSTEM FOR PRODUCING OLEFIN BY STEAM CRACKING HEAVY HYDROCARBON**

(30) Priority: 25.07.2022 CN 202210878593
(71) Applicant: China Petroleum & Chemical Corporation, Beijing 100728 (CN); Sinopec Engineering Incorporation, Beijing 100101 (CN)
(72) Inventor: HE, Xiou, Beijing 100101 (CN); WU, Defei, Beijing 100101 (CN); FAN, Chuanhong, Beijing 100101 (CN); LIN, Jiangfeng, Beijing 100101 (CN); WANG, Zizong, Beijing 100728 (CN); ZHAO, Yonghua, Beijing 100101 (CN); BAI, Fei, Beijing 100101 (CN); SHAO, Chen, Beijing 100101 (CN); XIAO, Jia, Beijing 100101 (CN); SHI, Yu, Beijing 100101 (CN)
(74) Representative: karo IP
(86) International application number: PCT/CN2023/109009
(87) International publication number: WO 2024/022308

(57) **Abstract**

The present application relates to a process and a system for producing olefins by steam cracking of a heavier hydrocarbon. The process for producing olefins by steam cracking of a heavier hydrocarbon comprises the steps of: subjecting a heavier hydrocarbon to a first separation to obtain a first vapour stream and a first liquid stream, partially condensing (second separation) the first vapour stream to obtain a condensate (third heavier fraction), introducing at least a portion of the third heavier fraction into the first liquid stream to obtain a first modified liquid stream, subjecting the first modified liquid stream to a third separation to obtain a second vapour stream and a second liquid stream, and carrying out steam cracking on the second vapour stream to obtain a cracked gas effluent containing the olefins. Compared with the prior art, the process and system of the present application can inhibit the coking of the convection zone, and provide a high utilization rate of heavier hydrocarbon (crude oil) and a high ethylene yield.

## Description

### Technical Field

The present application relates to the technical field of petrochemical industry, particularly to a process and a system for producing olefin by steam cracking of a heavier hydrocarbon.

### Background Art

Compared with the traditional cracking feedstock, when used as the feedstock of steam cracking furnace, heavier hydrocarbons (crude oil and the like) have the problems of high final boiling point (higher than 520 °C), containing impurities such as resin, asphaltene and the like, difficult vaporization, easy coking and the like, so that the design of the cracking furnace and the process of production need to be modified and improved correspondingly to adapt to the characteristics of the heavier hydrocarbons (crude oil and the like).

CN111196936A discloses a combined processing method and plant for producing olefin by direct cracking of crude oil, which is used for producing olefin directly from crude oil, and comprises removing impurities such as water, metals, and non-metals in the feed by pretreatment such as desalting and dewatering, then feeding the treated feed into the first tube bank in convection zone of an ethylene cracking furnace for heating, feeding the heated feed into a vapour-liquid separator, feeding the separated diesel oil and lighter gaseous hydrocarbon into the second tube bank of the convection zone and a radiant zone for steam cracking reaction to produce olefin. The liquid from the vapour-liquid separator contains components such as atmospheric residue and the like, and is sent to a hydrogenation unit for further cracking, hydrogenated tail oil, light naphtha and the like generated by cracking are sent back to the second tube bank of the convection zone as feedstock for ethylene cracking, and other generated fractions are sent to the outside unit as byproducts or other raw materials.

CN107001955A discloses a system and method for efficiently cracking hydrocarbon mixtures, such as mixtures comprising compounds with a standard boiling temperature of greater than 450 °C, 500 °C or even greater than 550 °C, such as whole crude oil.

CN101528894A discloses a method of processing a liquid crude oil and/or natural vapour condensate feedstock, comprising: subjecting the feedstock to an evaporation step to form a vapor product and a liquid product, subjecting the vapor product to severe thermal cracking, and subjecting the liquid product to a crude oil refinery process.

CN 101778929A discloses a method for processing a feedstock containing condensate and crude oil in an olefin production plant. The feedstock is vaporized and separated into gaseous and liquid hydrocarbons. The gaseous hydrocarbon stream is thermally cracked in the plant. Liquid hydrocarbons are recovered.

CN 103210063A discloses a process for cracking heavier hydrocarbon feed. The heavier hydrocarbon feed is passed to a first zone of a vaporization unit to separate a first vapor stream and a first liquid stream. The first liquid stream is passed to a second zone of the vaporization unit and intimately contacted with a counter-current steam to produce a second vapor stream and a second liquid stream. The second vapor stream is contacted with a wash liquid in a rectification section to form a rectified stream. The first vapor stream and the rectified stream are cracked in the radiant zone of the steam cracker to produce a cracked effluent.

### Summary of the Invention

The inventors of the present application found through diligent research that it is very important to control the concentration of heavier hydrocarbons during evaporation and to prevent coking in the convection zone and the radiant zone when direct steam cracking of a heavier hydrocarbon is employed, since the fraction of heavier hydrocarbons (crude oil, etc.) is wide and the content of heavier hydrocarbons is high. In the prior art, when the crude oil is treated by adopting a multi-stage flash method, non-volatile components are enriched in a first-stage liquid phase, so that the content of non-volatile components in the first-stage flash liquid phase is high, and the direct entering of the non-volatile components into the convection zone may easily cause a coking of the convection zone; in the prior art, when the potential aromatic content in the first-stage flash vapor phase is high, energy waste and reduction of ethylene yield may be caused by direct steam cracking; the first-stage flash vapor phase in the prior art may easily carry non-volatile components, and direct steam cracking will result in coking in the radiant zone.

Therefore, the present application aims to provide a process and a system for producing olefin by steam cracking of a heavier hydrocarbon, which can simplify the atmospheric and vacuum distillation unit in traditional methods and systems, enable a simple operation and a flexible splitting of heavier hydrocarbon, balance the feedstock supply of ethylene cracking unit and oil refining related processing unit such as reforming unit, simplify the vapour-liquid separation process, and realize short-process reconstruction of the process, and in which the vapour-liquid separation process has a high efficiency, and can effectively solve the defects of multistage flash process in the prior art, solve the problems of impurity entrainment in heavy components during the vapour-liquid separation process, utilization of aromatic hydrocarbons and coking reduction, realize long-period operation of the cracking furnace, further reduce the energy consumption of the ethylene plant and has wide adaptability to crude oil.

Specifically, the present application relates to the following aspects.
1. A process for producing olefins by steam cracking of a heavier hydrocarbon, comprising the steps of:
   subjecting a heavier hydrocarbon to a first separation (such as flash) to obtain a first vapour stream and a first liquid stream,
   subjecting the first vapour stream to partial condensation (such as fractional distillation, referred to as the second separation) to obtain a condensate (referred to as the third heavier fraction, preferably having an initial boiling point of 165 °C or higher, more preferably having a distillation range of 180-380 °C),
   introducing at least a portion (e.g., 50wt% or higher or 80wt% of higher of the total weight) of the third heavier fraction into the first liquid stream to obtain a first modified liquid stream,
   subjecting the first modified liquid stream to a third separation (e.g., flash) to obtain a second vapour stream and a second liquid stream, and
   subjecting the second vapour stream to steam cracking to obtain a cracked product containing the olefin.
2. The process according to any one of the preceding or subsequent aspects, wherein the final boiling temperature of the heavier hydrocarbon is 540 °C or higher (preferably the initial boiling temperature is 15 °C and the final boiling temperature is 750 °C or higher), and/or the API index of the heavier hydrocarbon is not less than 32 (preferably 38 or more), and/or the content of non-volatile components of the heavier hydrocarbon is 25 wt% or less (preferably 0.1 to 5.5 wt%), based on the total weight of the heavier hydrocarbon, and/or the potential aromatic content of the heavier hydrocarbon is 10 to 40 wt%, based on the total weight of the heavier hydrocarbon.
3. The process according to any one of the preceding or subsequent aspects, wherein the heavier hydrocarbon is at least one selected from the group consisting of paraffinic base crude oil, intermediate base crude oil, naphthenic base crude oil, condensate oil, and refined products.
4. The process according to any one of the preceding or subsequent aspects, wherein the heavier hydrocarbon is preheated to a temperature of 200-400 °C (preferably 240-300 °C) prior to the first separation.
5. The process according to any one of the preceding or subsequent aspects, wherein the first vapour stream has a content of non-volatile components of 0.5 wt% or less, based on the total weight of the first vapour stream.
6. The process according to any one of the preceding or subsequent aspects, wherein the first vapour stream is subjected to the second separation to obtain at least a third lighter fraction (preferably having a final boiling temperature of 90 °C or lower), a first intermediate fraction (preferably having a distillation range of 80-170 °C) and the third heavier fraction.
7. The process according to any one of the preceding or subsequent aspects, further comprising subjecting the first intermediate fraction to at least one treatment selected from reforming treatment, refinery processing, and isomeric separation.
8. The process according to any one of the preceding or subsequent aspects, wherein the third heavier fraction has a content of non-volatile components of 1 wt% or less (preferably 0.5 wt% or less), based on the total weight of the third heavier fraction.
9. The process according to any one of the preceding or subsequent aspects, wherein the weight ratio of the third heavier fraction to the first liquid stream is 5-25% (preferably 15% - 18%).
10. The process according to any one of the preceding or subsequent aspects, wherein the first modified liquid stream is subjected to first mixing with a primary (or a first stage) dilution steam to obtain a first mixed stream, then the first mixed stream is subjected to first heating, then the first mixed stream subjected to the first heating is subjected to second mixing with a secondary (or a second stage) dilution steam to obtain a second mixed stream, and then the second mixed stream is subjected to the third separation to obtain the second vapour stream and the second liquid stream.
11. The process according to any one of the preceding or subsequent aspects, wherein the temperature of the primary dilution steam is 180-400 °C, preferably 200-350 °C, and/or the temperature of the first mixed stream after the first heating is 400 °C or lower, preferably 250-350 °C, and/or the temperature of the secondary dilution steam is 400-630 °C, preferably 450-600 °C, and/or the weight ratio of the first modified liquid stream to the primary dilution steam is 1: (0.1-0.5), preferably 1: (0.2-0.4), and/or the weight ratio of the first modified liquid stream to the secondary dilution steam is 1: (0.2-0.8), preferably 1: (0.3-0.65) and/or the temperature of the second mixed stream is 280-360 °C (preferably 310-340 °C).
12. The process according to any one of the preceding or subsequent aspects, wherein a liquid hydrocarbon is further introduced into the first liquid stream, and/or the first modified liquid stream, and/or the first mixed stream, in a weight ratio of the liquid hydrocarbon to the first liquid stream of (0.1-0.85): 1 (preferably (0.15-0.3): 1).
13. The process according to any one of the preceding or subsequent aspects, wherein in the first separation the vaporization rate of the heavier hydrocarbon (referred to as the first vaporization rate) is 15-60%, preferably 25-45%, and in the third separation the vaporization rate of the second mixed stream (referred to as the second vaporization rate) is 40-80%, preferably 50-75%.
14. The process according to any one of the preceding or subsequent aspects, wherein the difference (in absolute value) between the first and second vaporization rates is 15% to 40% (preferably 20% to 30%).
15. The process according to any one of the preceding or subsequent aspects, wherein the first modified liquid stream or the second mixed stream is subjected to the third separation to obtain a vapour stream (referred to as primary vapour stream) and a liquid stream (referred to as primary liquid stream), and at least a portion (for example, 50wt% or less or 20wt% or less of the total weight) of the third heavier fraction and/or a separation aid (also referred to as cooling aid) is brought into counter-current contact with the primary vapour stream to obtain the second vapour stream.
16. The process according to any one of the preceding or subsequent aspects, wherein the weight ratio of the second vapour stream to the third heavier fraction is 1: (0.01-0.35), preferably 1: (0.05-0.2), and/or the weight ratio of the second vapour stream to the separation aid is 1: (0.01-0.2), preferably 1: (0.05-0.15).
17. The process according to any one of the preceding or subsequent aspects, wherein the primary vapour stream is first contacted with the separation aid in a counter-current manner, and then contacted with the third heavier fraction in a counter-current manner, or the primary vapour stream is first contacted with the third heavier fraction in a counter-current manner, and then contacted with the separation aid in a counter-current manner.
18. The process according to any one of the preceding or subsequent aspects, wherein the separation aid is at least one selected from the group consisting of liquid hydrocarbons and liquid water.
19. The process according to any one of the preceding or subsequent aspects, wherein the primary vapour stream is first contacted with the liquid water in a counter-current manner, and then contacted with the liquid hydrocarbon in a counter-current manner, or the primary vapour stream is first contacted with the liquid hydrocarbon in a counter-current manner, and then contacted with the liquid water in a counter-current manner, or the primary vapour stream is contacted with both the liquid water and the liquid hydrocarbon at the same time in a counter-current manner.
20. The process according to any one of the preceding or subsequent aspects, wherein the final boiling point of the liquid hydrocarbon is 200-540 °C (preferably 250-450 °C or 300-420 °C), and the liquid hydrocarbon is preferably at least one selected from heavy naphtha, aviation kerosene and diesel oil, based on the total weight of the liquid hydrocarbon.
21. The process according to any one of the preceding or subsequent aspects, wherein the second vapour stream is subjected to a second heating to a temperature of 500-600 °C (preferably 510-550 °C) prior to the steam cracking, and the content of non-volatile components of the second vapour stream is 1 wt% or less (preferably 0.5 wt% or less), based on the total weight of the second vapour stream.
22. A system for producing olefins by steam cracking of a heavier hydrocarbon, comprising:
   a first vapour-liquid separator (8) configured to enable a first separation (such as flash) of the heavier hydrocarbon, resulting in a first vapour stream and a first liquid stream,
   a separator, such as a separation column (7), configured to partially condense (e.g. by fractional distillation, referred to as a second separation) the first vapour stream to obtain a condensate (referred to as a third heavier fraction, preferably having an initial boiling temperature of 165 °C or higher, more preferably having a distillation range of 180-380 °C),
   a introduction means configured to introduce at least a portion (such as 50 wt% or higher or 80 wt% or higher of the total weight) of the third heavier fraction into the first liquid stream to obtain a first modified liquid stream,
   a second vapour-liquid separator (9) configured to enable a third separation (such as flash) of the first modified liquid stream to obtain a second vapour stream and a second liquid stream,
   a steam cracker configured to conduct steam cracking on the second vapour stream to obtain a cracked product comprising the olefins.

In another aspect, the present application also relates to the following aspects.
1. A process for producing olefins by steam cracking of a heavier hydrocarbon, comprising the steps of:
   S1, feeding a preheated heavier hydrocarbon into a first vapour-liquid separator (8) for first separation to obtain a first vapour stream and a first liquid stream;
   S2, performing steam cracking on at least a portion of the first liquid stream to obtain a cracked product comprising olefin; sending at least a portion of the first vapour stream to a separation column (7) for second separation to obtain at least a third lighter fraction, a first intermediate fraction and a third heavier fraction, and subjecting the first intermediate fraction to a post-treatment, wherein the post-treatment does not comprise steam cracking.
2. The process according to any one of the preceding or subsequent aspects, wherein step S2 further comprises:
   performing a first mixing of at least a portion of the first liquid stream with a primary dilution steam to obtain a first mixed stream; subjecting the first mixed stream to a first heating, and then performing a second mixing of the heated first mixed stream with a superheated secondary dilution steam to obtain a second mixed stream;
   passing the second mixed stream into a second vapour-liquid separator (9) for a third separation to obtain a second vapour stream and a second liquid stream; and
   subjecting the second vapour stream to a second heating, and then passing it into a radiant zone of a steam cracker for steam cracking to obtain a cracked product comprising olefin.
3. The process according to any one of the preceding or subsequent aspects, wherein in step S1, the temperature of the preheated heavier hydrocarbon is 200-400 °C, preferably 240-370 °C;
   optionally, the first vapour stream comprises a first lighter fraction and the first liquid stream comprises a first heavy component;
   optionally, the preheated heavier hydrocarbon has a vaporization rate of 20-65%, preferably 30-50%.
4. The process according to any one of the preceding or subsequent aspects, wherein in step S2, the weight ratio of the first liquid stream to the primary dilution steam is 1: (0.1-0.5), preferably 1: (0.2-0.4);
   optionally, the post-treatment comprises one or more of reforming treatment, oil refining, and isomeric separation;
   optionally, the process further comprises: in the step S2, passing at least a portion of the primary dilution steam into a third heating section (3A) for heating to obtain superheated primary dilution steam, and performing the first mixing of the superheated primary dilution steam with the first liquid stream;
   optionally, the temperature of the superheated primary dilution steam is 180-400 °C, preferably 200-350 °C;
   optionally, the temperature of the first mixed stream is 150-350 °C, preferably 190-300 °C;
   optionally, the temperature of the first mixed stream after the first heating is 400 °C or lower, preferably 250-350 °C;
   optionally, the temperature of the superheated secondary dilution steam is 400-630 °C, preferably 450-600 °C;
   optionally, the weight ratio of the first liquid stream to the secondary dilution steam is 1: (0.2-0.8); preferably 1: (0.3-0.65).
5. The process according to any one of the preceding or subsequent aspects, wherein the second vapour stream comprises entrained steam and a second lighter fraction; the second liquid stream comprises a second heavier fraction; the final boiling temperature of the second lighter fraction is 330-480 °C; the initial boiling temperature of the second heavier fraction is not higher than the final boiling point of the second lighter fraction;
   optionally, step S2 further comprises:
   introducing at least a portion of the third heavier fraction and/or a cooling aid into the first heavy component;
   introducing at least a portion of the third heavier fraction and/or a separation aid into the vapour-phase space of the second vapour-liquid separator (9) and contacting it with the second vapour stream counter-currently ;
   optionally, subjecting at least a portion of the third heavier fraction to steam cracking to obtain a product comprising olefins;
   optionally, the weight ratio of the second mixed vapour stream to the third heavier fraction is 1: (0.01-0.2), preferably 1: (0.05-0.15);
   optionally, the cooling aid is one selected from liquid hydrocarbon and/or water or both;
   optionally, the separation aid is one selected from liquid hydrocarbon and/or water or both;
   optionally, the weight ratio of the second mixed vapour stream to the water is 1: (0.01-0.2), preferably 1: (0.05-0.15);
   optionally, the weight ratio of the second mixed vapour stream to the liquid hydrocarbon is 1: (0.01-0.2), preferably 1: (0.05-0.15);
   optionally, the process further comprises: introducing the separation aid at least one mode selected from the group consisting of the following: introducing water at a position above the liquid hydrocarbon along the axial direction of the second vapour-liquid separator (9); or introducing water at a position below the liquid hydrocarbon along the axial direction of the second vapour-liquid separator (9); or introducing water at a position in the same plane as the liquid hydrocarbon along the axial direction of the second vapour-liquid separator (9).
6. The process according to any one of the preceding or subsequent aspects, wherein the steam cracker comprises a convection zone and a radiant zone; the convection zone comprises a first heating section (1A), an optional heavier hydrocarbon preheating section (1B), an optional second heating section (2), a third heating section (3A), a fourth heating section (4), a fifth heating section (3B) and a sixth heating section (5) along the height direction of the steam cracker;
   optionally, step S1 further includes:
   passing the heavier hydrocarbon to be preheated into the first heating section (1A) for first preheating to obtain a first preheated heavier hydrocarbon; respectively passing at least a portion of the first preheated heavier hydrocarbon and a heat exchange medium into a heat exchanger (6A) for heat exchange to obtain a second preheated heavier hydrocarbon; then passing the second preheated heavier hydrocarbon as the preheated heavier hydrocarbon into the first vapour-liquid separator (8);
   optionally, the heat exchange medium comprises steam heated by a heater (6B), or steam heated by the second heating section (2), or steam from a steam source; optionally, the temperature of the steam is 250-450 °C; or
   introducing at least a portion of the first preheated heavier hydrocarbon into a heater (6B) for heating to obtain a third preheated heavier hydrocarbon; then passing the third preheated heavier hydrocarbon as the preheated heavier hydrocarbon into the first vapour-liquid separator (8); or
   passing at least a portion of the first preheated heavier hydrocarbon from the first heating stage (1A) to the heavier hydrocarbon preheating stage (1B) for heating, to obtain a fourth preheated heavier hydrocarbon; passing the fourth preheated heavier hydrocarbon as the preheated heavier hydrocarbon into the first vapour-liquid separator (8);
   optionally, the first preheated heavier hydrocarbon is desalted prior to the heat exchange and/or heating, or the heavier hydrocarbon to be preheated is desalted prior to the first preheating.
7. A system for producing olefins by steam cracking of a heavier hydrocarbon, the system comprising a steam cracker, a separation column (7) and a first vapour-liquid separator (8); the steam cracker comprises a convection zone and a radiant zone;
   the first vapour-liquid separator (8) is configured to enable the preheated heavier hydrocarbon to enter the first vapour-liquid separator for first separation, to obtain a first vapour stream and a first liquid stream;
   the radiant zone of the steam cracker is configured to conduct steam cracking on at least a portion of the first liquid stream to obtain a cracked product comprising olefins;
   the separation column (7) is configured to send at least a portion of the first vapour stream to the separation column (7) for second separation to obtain at least a third lighter fraction, a first intermediate fraction and a third heavier fraction;
   the system further comprises a post-treatment device configured to conduct a post-treatment on the first intermediate fraction, which does not comprise a steam cracker.
8. The system according to any one of the preceding or subsequent aspects, wherein the convection zone is provided with a feedstock heating inlet and a feedstock heating outlet; the radiant zone is provided with a steam cracking inlet and a cracked gas effluent outlet;
   the first vapour-liquid separator (8) is provided with a vapour-liquid separation inlet, an optional third heavier fraction circulation inlet, an optional cooling aid inlet, a first vapour stream outlet and a first liquid stream outlet; the vapour-liquid separation inlet of the first vapour-liquid separator (8) is communicated with the feedstock heating outlet of the convection zone; the first liquid stream outlet of the first vapour-liquid separator (8) is communicated with the steam cracking inlet of the radiant zone;
   the cooling aid inlet comprises a liquid hydrocarbon inlet and/or a water inlet;
   the separation column (7) is provided with a first vapour stream inlet, and a third lighter fraction outlet, a first intermediate fraction outlet and a third heavier fraction outlet arranged sequentially from top to bottom along the height direction of the separation column (7); the first vapour stream inlet of the separation column (7) is communicated with the first vapour stream outlet of the first vapour-liquid separator (8);
   optionally, the system further comprises a second vapour-liquid separator (9);
   the second vapour-liquid separator (9) is provided with a second mixed stream inlet, a second vapour stream outlet, a second liquid stream outlet, an optional third heavier fraction inlet and an optional separation aid inlet;
   the separation aid inlet comprises a liquid hydrocarbon inlet and/or a water inlet;
   the separation aid inlet is positioned above the second mixed stream inlet along the axial direction of the second vapour-liquid separator (9);
   wherein the first liquid stream outlet of the first vapour-liquid separator (8) is communicated with the second mixed stream inlet of the second vapour-liquid separator (9); a primary dilution steam inlet and a superheated secondary dilution steam inlet are provided on a communicating pipeline between the second mixed stream inlet of the second vapour-liquid separator (9) and the first liquid stream outlet of the first vapour-liquid separator (8); the primary dilution steam inlet is located at the upstream of the superheated secondary dilution steam inlet along the flow direction of the stream; optionally, the third heavier fraction outlet of the separation column (7) is respectively communicated with the third heavier fraction circulation inlet of the first vapour-liquid separator (8) and the third heavier fraction inlet of the second vapour-liquid separator (9); and
   the second vapour stream outlet of the second vapour-liquid separator (9) is communicated with the steam cracking inlet of the radiant zone.
9. The system according to any one of the preceding or subsequent aspects, wherein the convection zone comprises a first heating section (1A), an optional heavier hydrocarbon preheating section (1B), an optional second heating section (2), a third heating section (3A), a fourth heating section (4), a fifth heating section (3B), and a sixth heating section (5) along the height direction of the steam cracker;
   wherein the heating inlet of the first heating section (1A) is used for introducing a heavier hydrocarbon to be preheated; the heating outlet of the first heating section (1A) is communicated with the vapour-liquid separation inlet of the first vapour-liquid separator (8); preferably, a pressure regulating valve is provided on an inlet pipeline of the vapour-liquid separation inlet of the first vapour-liquid separator (8) and used for controlling the preheating temperature of the heavier hydrocarbon entering the first vapour-liquid separator (8); optionally, a pressure regulating valve is provided on the outlet pipeline of the first vapour stream outlet to further control the vaporization rate;
   a heating inlet of the fourth heating section (4) is communicated with the first liquid stream outlet of the first vapour-liquid separator (8) through a first pipeline; a heating inlet of the third heating section (3A) is communicated with a primary steam source; a heating outlet of the third heating section (3A) is connected to the first pipeline;
   a heating outlet of the fourth heating section (4) is communicated with the second mixed stream inlet of the second vapour-liquid separator (9) through a second pipeline; a heating inlet of the fifth heating section (3B) is communicated with a secondary steam source; a heating outlet of the fifth heating section (3B) is connected to the second pipeline;
   a heating inlet of the sixth heating section (5) is communicated with a second vapour stream outlet of the second vapour-liquid separator (9); a heating outlet of the sixth heating section (5) is communicated with the steam cracking inlet of the radiant zone;
   optionally, the second liquid stream outlet of the second vapour-liquid separator (9) may be communicated with a hydrocracking or catalytic cracking unit;
   optionally, the second vapour-liquid separator (9) is internally provided with a tray, a hydrocyclone or a combined structure of the two.
10. The system according to any one of the preceding or subsequent aspects, wherein the system further comprises a heavier hydrocarbon preheating unit comprising a heavier hydrocarbon heating inlet and a heavier hydrocarbon heating outlet; the heavier hydrocarbon heating inlet is communicated with the heating outlet of the first heating section (1A), and the heavier hydrocarbon heating outlet is communicated with the vapour-liquid separation inlet of the first vapour-liquid separator (8);
   optionally, the heavier hydrocarbon preheating unit comprises a heat exchanger (6A) and an optional heater (6B); the heat exchanger (6A) comprises a first heating inlet for heavier hydrocarbon, a heat exchange medium inlet, a first heating outlet for heavier hydrocarbon and an outlet for heat exchange medium after heat exchange; the first heating inlet for heavier hydrocarbon is formed as a heavier hydrocarbon heating inlet of the heavier hydrocarbon preheating unit and is communicated with the heating outlet of the first heating section (1A); the first heating outlet for heavier hydrocarbon is formed as a heavier hydrocarbon heating outlet of the heavier hydrocarbon preheating unit and is communicated with the vapour-liquid separation inlet of the first vapour-liquid separator (8);
   the heater (6B) comprises a first heating inlet for heat exchange medium and a first heating outlet for heat exchange medium; the second heating section (2) comprises a second heating inlet for heat exchange medium and a second heating outlet for heat exchange medium; the first heating inlet for heat exchange medium and the second heating inlet for heat exchange medium are respectively communicated with a steam source;
   a heat exchange medium inlet of the heat exchanger (6A) is communicated with the first heating outlet for heat exchange medium of the heater (6B), or a heat exchange medium inlet of the heat exchanger (6A) is communicated with the second heating outlet for heat exchange medium of the second heating section (2); or the heat exchange medium inlet of the heat exchanger (6A) is communicated with a steam source;
   or the heavier hydrocarbon preheating unit comprises a heater (6B); the heater (6B) comprises a second heating inlet for heavier hydrocarbon and a second heating outlet for heavier hydrocarbon; the second heating inlet for heavier hydrocarbon is formed as a heavier hydrocarbon heating inlet of the heavier hydrocarbon preheating unit and is communicated with the heating outlet of the first heating section (1A); the second heating outlet for heavier hydrocarbon is formed as a heavier hydrocarbon heating outlet of the heavier hydrocarbon preheating unit and is communicated with the vapour-liquid separation inlet of the first vapour-liquid separator (8);
   optionally, the heating outlet of the first heating section (1A) is communicated with a heating inlet of the heavier hydrocarbon preheating section (1B); a heating outlet of the heavier hydrocarbon preheating section (1B) is communicated with the vapour-liquid separation inlet of the first vapour-liquid separator (8) through a third pipeline;
   optionally, the heat exchanger (6A) or the heater (6B) is connected to a third pipeline.

### Technical effects

Compared with the prior art, the process and the system provided by the present application can achieve one of the following technical effects or the combination of all or part of the following technical effects:
(1) The first intermediate fraction can be reasonably processed and used for a reforming unit or used as cracking feed, and is particularly suitable for feedstock with a high potential aromatic content when used for reforming unit, so that when a two-stage separation is performed and the first vapour stream is separated, having high flexibility of heavier hydrocarbons (crude oils), and the heavier hydrocarbon can be split according to the properties of components thereof with different distillation ranges, so that a high-efficiency utilization of components with different distillation ranges of the heavier hydrocarbon, e.g. as a feedstock for cracking or reforming, and the like, can be realized, aromatic hydrocarbon does not participate in steam cracking, the energy utilization efficiency is higher, and the ethylene yield is higher.
(2) The third heavier fraction is introduced into the first liquid stream, so that the specification of the first liquid stream can be effectively regulated, the temperature of the first liquid stream can be controlled, coking in the preheating and vaporization process of the heavier hydrocarbon in the fourth heating section can be effectively inhibited, a long-period operation of the cracking furnace can be ensured, and meanwhile, the vaporization rate of the heavier hydrocarbon can be effectively regulated according to the specification of the heavier hydrocarbon, so that the temperature of the stream subjected to the second flash can be higher, the vaporization rate of the second flash can be higher, more second vapour feedstock suitable for cracking can be effectively separated, the utilization rate of the heavier hydrocarbon (crude oil) is high, and the ethylene yield is high;
(3) The introduction of the separation aid can reduce the entering of the non-ideal components for cracking to the radiant zone, avoid the coking of in the furnace tube of the radiant zone, prolong the operation period and improve the energy utilization efficiency;
(4) The first stage flash no longer poses a requirement on or concerns about entrainment of non-volatile components, thereby reducing equipment complexity. The first vapour phase is separated, the third heavier fraction is merged into the first liquid stream, so that the non-volatile component (if entrained) contained in the third heavier fraction can be introduced into the first liquid stream, the problem of enrichment of non-volatile component in the first liquid stream can be alleviated, the specification of the first liquid stream can be effectively regulated, the temperature of the first liquid stream can be controlled, coking in the preheating and vaporization process of the heavier hydrocarbon in the fourth heating section can be effectively inhibited, and the separation efficiency of the second flash is improved.
(5) By adopting the solution of the present application, the second vapour stream comprises little or no naphtha fraction (having a dry point of less than or equal to 180 °C), the distillation range of its fraction is narrow, the selection of its cracking conditions such as COT can balance the efficient cracking of all components, the olefin yield is high, and the energy consumption is low.

### Brief Description of the Drawings

Fig. 1 is an exemplary flow diagram for the steam cracking of a heavier hydrocarbon to produce olefins, as provided by the present application.
Fig. 2 is an exemplary flow diagram for the steam cracking of a heavier hydrocarbon to produce olefins, as provided by the present application.
Fig. 3 is an exemplary flow diagram for the steam cracking of a heavier hydrocarbon to produce olefins, as provided by the present application.
Fig. 4 is an exemplary flow diagram for producing olefins by steam cracking of a heavier hydrocarbon, as provided by the present application.
Fig. 5 is an exemplary flow diagram for producing olefins by steam cracking of a heavier hydrocarbon, as provided by the present application.
Fig. 6 is a comparison of TBP curves before and after upgrading of heavier hydrocarbon components provided by the present application.
Fig. 7 is a comparison of TBP curves before and after upgrading of heavier hydrocarbon components provided by the present application.

### Description of the reference numerals

1A - first heating section;
BFW - boiler feed water preheating section;
1B - heavier hydrocarbon preheating section;
2 - second heating section;
3A - third heating zone;
4 - fourth heating section;
VHS - hyperpressure steam superheating section;
3B - fifth heating section;
5 - sixth heating section;
6A - heat exchanger;
6B - heater;
7 - separation column;
8 - first vapour-liquid separator; 9 - second vapour-liquid separator.

### Detailed Description of the Invention

The present application will be illustrated in detail hereinbelow with reference to embodiments thereof, but it should be noted that the scope of the present application is not limited by those embodiments, but is defined by the appended claims.

All publications, patent applications, patents, and other references cited herein are incorporated by reference in their entirety. Unless defined otherwise, all technical and scientific terms used herein have the same meaning as commonly understood by those skilled in the art. In case of conflict, the contents described herein, including definitions, should prevail.

Where a material, substance, method, step, device, component, or the like is described herein as "commonly known to those skilled in the art", "prior art" or the like, it is to be understood that said material, substance, method, step, device and component cover not only those conventionally used in the art at the time of filing the present application, but also those not commonly used at present but will become commonly known in the art to be suitable for a similar purpose.

Throughout the context of the specification and claims, unless explicitly stated otherwise, the term "comprise" or its variations such as "comprises" or "comprising", etc., shall be understood as including the element or component explicitly mentioned, without excluding any other element or component.

In the context of the present application, spatially relative terms, such as "lower," "below," "beneath," "upper," "above," "over," and the like, may be used, for convenience, to describe the relationship between one element or feature and another element or feature in the figures. It can be appreciated that the spatially relative terms are intended to cover different orientations of the article in use or operation in addition to that shown in the figures. For example, if the article shown in the figures is turned over, an element described as "below" or "beneath" another element or feature would then be oriented "above" the element or feature. Thus, the exemplary term "below" may cover both the orientation of "below" and the orientation of "above". The article may be otherwise oriented (e.g., rotated 90 degrees or at other orientations) and the spatially relative terms used herein should be interpreted accordingly.

In the context of the present application, the terms "first", "second", and the like are used to distinguish two different elements or parts, while are not used to define a particular positional or relative relationship.

In the context of the present application, all numerical values of a parameter (e.g., quantity or condition) shall be understood as being modified in all instances by the term "about", no matter whether or not the term "about" is actually present before the numerical value.

In the context of the present application, the terms "primary dilution steam", "secondary dilution steam" and the like are used only to distinguish steams introduced in different steps, while do not have any actual meaning concerning the nature of the steam itself and the like.

In the context of the present application, various equipment used in the present application may have a structure conventionally used in the art, with no particular limitation.

In the context of the present application, heavier hydrocarbons refer to wide-cut mixed hydrocarbons having a final boiling point of generally above 540 °C, for example an initial boiling temperature of 15 °C and a final boiling temperature of above 750 °C. Particularly, the heavier hydrocarbons may be one or more selected from the following feedstock: paraffinic base crude oil, intermediate base crude oil, naphthenic base crude oil, condensate oil, refined products, and the like. Examples of the refined products include gasoline, kerosene, diesel oil, tail oil, fuel oil, and reformate from a refinery, that have been processed through a refinery unit such as atmospheric and vacuum, reforming, catalytic, and coking units.

In the context of the present application, the method for measuring the initial boiling point, the final boiling point and the distillation range is the atmospheric and vacuum distillation of crude oil, wherein the atmospheric distillation analysis is carried out in accordance with ASTM D-2892, and the vacuum distillation analysis is carried out in accordance with ASTM D-5236.

In the context of the present application, API refers to the specific gravity index, which is measured in accordance with ASTM D-2320.

In the context of the present application, non-volatile components refer to components having a final boiling point of more than 590 °C, and the analysis of non-volatile components is carried out in accordance with ASTM D6352.

In the context of the present application, the potential aromatic content is measured by atmospheric distillation, and the analysis is carried out in accordance with ASTM D-2892.

In the context of the present application, the vaporization rate refers to the mass percentage of the vapour stream (excluding steam) to the total amount of feed (excluding steam).

In the context of the present application, the potential aromatic content refers to the weight ratio of the sum of the amount of aromatics obtained by converting all naphthenes in the feed to aromatics and the amount of aromatics already present in the feed to the total amount of the feed.

In the context of the present application, unless specifically stated otherwise, all percentages, parts, ratios, etc. are expressed by weight and all pressures given are gauge pressures.

In the context of the present application, any two or more embodiments of the present application may be arbitrarily combined, and the resulting technical solution forms a part of the initial disclosure of the present application and falls within the scope of the present application.

In an embodiment of the present application, a process for producing olefins by steam cracking of a heavier hydrocarbon is provided. According to the present application, the process for producing olefins by steam cracking of a heavier hydrocarbon is carried out in the system for producing olefins by steam cracking of a heavier hydrocarbon described hereinafter. For this reason, contents that are not described in detail for the process may refer directly to relevant contents described below for the system, or vice versa.

According to an embodiment of the present application, the process for producing olefins by steam cracking of a heavier hydrocarbon comprises a step of subjecting a heavier hydrocarbon to a first separation to obtain a first vapour stream and a first liquid stream. Here, the first separation may be performed in any manner known in the art, and for example, any manner or method capable of separating the heavier hydrocarbon into a vapour phase and a liquid phase (vapour-liquid separation), particularly flash, may be mentioned.

According to an embodiment of the present application, the process for producing olefins by steam cracking of a heavier hydrocarbon further comprises a step of partially condensing (referred to as the second separation) the first vapour stream to obtain a condensate (referred to as the third heavier fraction). Here, the partial condensation or the second separation may be performed in any manner known in the art, and for example, any manner or method capable of further separating the first vapour stream by vapour-liquid separation after cooling into a plurality of components or fractions, particularly fractional distillation, may be mentioned. According to the present application, unlike the prior art, the first vapour stream is not sent directly to steam cracking.

According to an embodiment of the present application, the initial boiling temperature of the third heavier fraction is 165 °C or higher, and the distillation range is preferably 180-380 °C. Such a control is conducted mainly for a comprehensive consideration of providing feedstock for reforming, saving investment and energy consumption and modifying the first liquid stream. If the dry point of the third heavier fraction is less than 165 °C, it is indicated that the second separation is incomplete, and a portion of the reforming feed is lost. If the dry point of the third heavier fraction is more than 380 °C, the amount of the first vapour stream is increased, the diameter of the separation column and the number of plates of the second separation need to be increased, and then the investment and energy consumption will be increased. In addition, the distillation range of the third heavier fraction is 180-380 °C, which is suitable for the modification of the first liquid stream. If the dry point is too low, for example, less than 300 °C, the third heavier fraction is liable to be vaporized, and therefore the content of vaporized non-volatile components in the first liquid stream is not reduced, whereas if the dry point is more than 380 °C the investment for the first separation and the second separation is increased, and the energy consumption for the second separation is increased.

According to an embodiment of the present application, the process for producing olefins by steam cracking of a heavier hydrocarbon further comprises a step of introducing at least a portion of the third heavier fraction into the first liquid stream to obtain a first modified liquid stream. It is preferred here that 50 wt% or higher or 80 wt% or higher of the total weight of the third heavier fraction is introduced into the first liquid stream. By introducing 50 wt% or higher or 80 wt% or higher of the total weight of the third heavier fraction, the distillation range of the first modified liquid stream can be effectively changed, so that the amplitude of the change of the first modified liquid stream in the convection zone can be widened, the coking of the fourth heating section (4) can be effectively prevented, and the vaporization rate of the second mixed stream in the third separation can be effectively adjusted.

According to an embodiment of the present application, the process for producing olefins by steam cracking of a heavier hydrocarbon further comprises a step of subjecting the first modified liquid stream to a third separation to obtain a second vapour stream and a second liquid stream. Here, as the third separation, any manner or method capable of separating the first modified liquid stream into a vapour phase and a liquid phase (vapour-liquid separation), particularly flash, may be mentioned.

According to an embodiment of the present application, the process for producing olefins by steam cracking of a heavier hydrocarbon further comprises a step of subjecting the second vapour stream to steam cracking to obtain a cracked product containing the olefins.

According to an embodiment of the present application, the API value of the heavier hydrocarbon is not less than 32 (preferably 38 or more).

According to an embodiment of the present application, the non-volatile components of the heavier hydrocarbon are present in an amount of 25 wt% or less (preferably 0.1 to 5.5 wt%), based on the total weight of the heavier hydrocarbon.

According to an embodiment of the present application, the heavier hydrocarbon has a potential aromatic content of 10 to 20 wt%, based on the total weight of the heavier hydrocarbon. By using the process of the present application, the aromatic hydrocarbon in the fraction with a final boiling point of less than 180 °C can be used as the reforming feedstock.

According to an embodiment of the present application, the heavier hydrocarbon is preheated to a temperature of 200-400 °C (preferably 240-300 °C) prior to the first separation.

According to the present application, since the problem of entrainment of non-volatile components in the first vapour stream is no longer considered, there is no particular limitation on the final boiling point of the first vapour stream and the content of non-volatile components thereof, which can be flexibly determined by those skilled in the art according to the actual situation. Nevertheless, according to an embodiment of the present application, the final boiling point is normally not higher than 400 °C in view of the operating stability of the process. The non-volatile components are normally present in an amount of 1 wt% or less, based on the total weight of the first vapour stream.

According to an embodiment of the present application, the first vapour stream is subjected to the second separation to obtain at least a third lighter fraction, a first intermediate fraction and the third heavier fraction. The second separation is preferably carried out in a fractionation column, such as the separation column 7 described below. The present application is not particularly limited with respect to the implementation, method, and the like of the second separation, and those known in the art may be employed. As a result of the second separation, it is preferred that the third lighter fraction consists essentially of components from C₁ to C₆, generally has a final boiling temperature of 90 °C or less, and can be sent to other cracking furnaces for cracking, while the first intermediate fraction consists essentially of components from C₆ to C₁₀, generally has a distillation range from 80 to 170 °C, and can be sent to the reforming unit. It can be appreciated that the initial boiling point or the final boiling point of each fraction in the present application is a range value, and any temperature within the range value may be possible in the actual operation, and the fraction to be separated can be selected in the present application according to the actual production needs.

According to an embodiment of the present application, the process for producing olefins by steam cracking of a heavier hydrocarbon further comprises subjecting the first intermediate fraction to at least one treatment selected from the group consisting of reforming treatment, refinery processing, and isomeric separation. When the potential aromatic content in the first intermediate fraction is high, the potential aromatic content of the vapour stream entering the radiant zone can be reduced after the first intermediate fraction being treated in the present application, so that as compared with the existing first-stage flash and second-stage flash processes, the potential aromatic content of the vapour stream entering the radiant zone is lower, the corresponding olefin yield is higher, and the cracking energy consumption is lower.

According to an embodiment of the present application, the non-volatile component of the third heavier fraction is present in an amount of 1 wt% or less (preferably 0.5 wt% or less), based on the total weight of the third heavier fraction.

According to an embodiment of the present application, the weight of the third heavier fraction and the first liquid stream is determined according to the nature of the heavier hydrocarbon. The principle is that the third heavier fraction can be used for adjusting the vaporization rate of the stream entering the second vapour-liquid separator and controlling the preheating temperature of the first modified liquid stream, so that coking in the preheating and vaporization process of heavier hydrocarbon in the fourth heating section (4) can be effectively inhibited, where the proportion is low, the adjustment is not significant, the control effect is poor, either the preheating temperature cannot be controlled, or the reduction of non-volatile components is not significant; and where the proportion is too high, the energy consumption is increased, the vaporization temperature is high and coking is easy to occur, or the vaporization rate may be lowered when the temperature is reduced to avoid coking.

According to the present application, the third heavier fraction can be used to regulate the vaporization rate of the stream entering the second vapour-liquid separator, where the proportion is low, the adjustment is not significant, and where the proportion is too high, the energy consumption is increased.

According to an embodiment of the present application, the first modified liquid stream is subjected to first mixing with a primary dilution steam to obtain a first mixed stream, then the first mixed stream is subjected to first heating, then the first mixed stream after the first heating is subjected to second mixing with a secondary dilution steam to obtain a second mixed stream, and then the second mixed stream is subjected to the third separation to obtain the second vapour stream and the second liquid stream.

According to an embodiment of the present application, the temperature of the primary dilution steam is 180-400 °C and preferably 200-350 °C.

According to an embodiment of the present application, the temperature of the first mixed stream after the first heating is 400 °C or less, preferably 250-350 °C.

According to an embodiment of the present application, the temperature of the secondary dilution steam is 400-630 °C, preferably 450-600 °C.

According to an embodiment of the present application, the weight ratio of the first modified liquid stream to the primary dilution steam is 1: (0.1-0.5), preferably 1: (0.2-0.4).

According to an embodiment of the present application, the weight ratio of the first modified liquid stream to the secondary dilution steam is 1: (0.2-0.8), preferably 1: (0.3-0.65).

According to an embodiment of the present application, the temperature of the second mixed stream is 320-400 °C.

According to an embodiment of the present application, a liquid hydrocarbon is further introduced into the first liquid stream, and/or the first modified liquid stream, and/or the first mixed stream.

According to an embodiment of the present application, the weight ratio of the liquid hydrocarbon to the first liquid stream is 1: (0.45-0.85) (preferably 1: (0.5-0.7)).

According to an embodiment of the present application, in the first separation, the vaporization rate of the heavier hydrocarbon (referred to as the first vaporization rate) is 15 to 60%, preferably 25 to 45%.

According to an embodiment of the present application, in the third separation, the vaporization rate of the first modified liquid stream or the second mixed stream (referred to as a second vaporization rate) is 40% to 80%, preferably 50% to 70%.

According to an embodiment of the present application, the difference (absolute value) between the first and second vaporization rates is between 15% and 50% (preferably between 30% and 40%). Such a control is conducted mainly for a comprehensive consideration of providing feedstock for reforming, saving the investment for the second separation and modifying the first liquid stream. For wide-cut crude oil, the first vaporization rate should not be too high, as long as the requirement for reforming feedstock can be met. The second vaporization rate can be increased to increase the chemical yield of the crude oil in view of satisfying the long-term safe operation, and thus the second vaporization rate is normally much higher than the first vaporization rate. If the first vaporization rate is higher than 50%, the second vaporization rate is normally lower than 80%, and the difference (absolute value) is lower than 30%, which may cause the second vaporization rate to lead to a dry point of the third heavier fraction of more than 380 °C, thus the aim of modifying the first liquid stream cannot be achieved, and the investment for the first separation and the second separation is increased, and the energy consumption of the second separation is increased. If the first vaporization rate is lower than 15%, the difference (absolute value) may be larger than 50%, the aromatic hydrocarbon material separated out by the second separation cannot meet the requirement for reforming feedstock, more light components are present in the second vapour stream, and the distillation range of the cracking feedstock is too wide, and consequently there is no appropriate cracking condition that can be used to achieve an efficient cracking.

According to an embodiment of the present application, the first modified liquid stream or the second mixed stream is subjected to the third separation to obtain a vapour stream (referred to as primary vapour stream) and a liquid stream (referred to as primary liquid stream), and at least a portion (such as 50 wt% or less or 20 wt% or less of the total weight) of the third heavier fraction is counter-currently contacted with the primary vapour stream to obtain the second vapour stream. By means of the countercurrent contact between the third heavier fraction and the primary vapour stream, the content of heavy components in the primary vapour stream can be effectively reduced, so that the requirement of cracking on the heavy components of the stream entering the radiant zone for cracking can be met, so as to reduce coking and prolong the operation cycle of the cracking furnace, and finally realize a cracking with low energy consumption and high efficiency.

According to an embodiment of the present application, the first modified liquid stream or the second mixed stream is subjected to the third separation to obtain a vapour stream (referred to as primary vapour stream) and a liquid stream (referred to as primary liquid stream), and a separation aid (also referred to as cooling aid) is counter-currently contacted with the primary vapour stream to obtain the second vapour stream. By means of the countercurrent contact between the separation aid and the primary vapour stream, the vaporization rate of heavier hydrocarbon can be effectively adjusted, and the content of heavy components in the second vapour stream can be effectively controlled, so that the requirement of cracking on the heavy components of the stream entering the radiant zone for cracking can be met, so as to reduce coking and prolong the operation cycle of the cracking furnace, and finally realize a cracking with low energy consumption and high efficiency.

According to an embodiment of the present application, the weight ratio of the second vapour stream to the third heavier fraction is 1: (0.01-0.35), preferably 1: (0.05-0.2).

According to an embodiment of the present application, the weight ratio of the second vapour stream to the separation aid is 1: (0.01-0.2), preferably 1: (0.05-0.15).

According to an embodiment of the present application, the primary vapour stream is first counter-currently contacted with the separation aid, and then counter-currently contacted with the third heavier fraction, or the primary vapour stream is first counter-currently contacted with the third heavier fraction, and then counter-currently contacted with the separation aid.

According to an embodiment of the present application, the separation aid is at least one selected from the group consisting of liquid hydrocarbons and liquid water. Here, as the liquid hydrocarbon, hydrocarbons having a final boiling point of 200-540 °C (preferably 250-450 °C or 300-400 °C) may be mentioned, preferably at least one selected from the group consisting of heavy naphtha, aviation kerosene oil.

According to an embodiment of the present application, the primary vapour stream is first counter-currently contacted with the liquid water, and then with the liquid hydrocarbon, or the primary vapour stream is first counter-currently contacted with the liquid hydrocarbon, and then with the liquid water, or the primary vapour stream is counter-currently contacted with the liquid water and the liquid hydrocarbon simultaneously.

According to an embodiment of the present application, the second vapour stream is subjected to a second heating to a temperature of 500-600 °C (preferably 510-500 °C) prior to the steam cracking.

According to an embodiment of the present application, the second vapour stream has a content of non-volatile components of 1 wt% or less (preferably 0.5 wt% or less), based on the total weight of the second vapour stream. If the content of non-volatile components of the second vapour stream does not meet this requirement, there may be a risk of coking in the radiant zone.

According to an embodiment of the present application, there is also provided a system for producing olefins by steam cracking of a heavier hydrocarbon, comprising: a first vapour-liquid separator (8) configured to perform a first separation on the heavier hydrocarbon, to obtain a first vapour stream and a first liquid stream; a separator, such as a separation column (7), configured to perform a second separation on the first vapour stream to obtain the third heavier fraction; a introduction means configured to introduce at least a portion of the third heavier fraction into the first liquid stream to obtain a first modified liquid stream; a second vapour-liquid separator (9) configured to perform a third separation on the first modified liquid stream, to obtain a second vapour stream and a second liquid stream; a steam cracker configured to conduct steam cracking on the second vapour stream to obtain a cracked product comprising the olefins.

According to an embodiment of the present application, the step S1 further comprises: passing the heavier hydrocarbon to be preheated into the first heating section for first preheating to obtain a first preheated heavier hydrocarbon; respectively passing at least a portion of the first preheated heavier hydrocarbon and a heat exchange medium into a heat exchanger for heat exchange to obtain a second preheated heavier hydrocarbon; then passing the second preheated heavier hydrocarbon into the first vapour-liquid separator as the preheated heavier hydrocarbon; wherein the heat exchange medium comprises steam heated by a heater, or steam heated by the second heating section, or steam from a steam source; optionally, the temperature of the steam is 250-450 °C.

According to an embodiment of the present application, the step S1 further comprises: introducing at least a portion of the first preheated heavier hydrocarbon into a heater for heating to obtain a third preheated heavier hydrocarbon; then passing the third preheated heavier hydrocarbon into the first vapour-liquid separator as the preheated heavier hydrocarbon.

According to an embodiment of the present application, the step S1 further comprises: passing at least a portion of the first preheated heavier hydrocarbon from the first heating section into the heavier hydrocarbon preheating section for heating to obtain a fourth preheated heavier hydrocarbon; passing the fourth preheated heavier hydrocarbon into the first vapour-liquid separator as the preheated heavier hydrocarbon.

According to an embodiment of the present application, the step S1 further comprises: subjecting the first preheated heavier hydrocarbon to desalting, and then to heat exchanging and/or heating, or subjecting the heavier hydrocarbon to be preheated to desalting, and then to first preheating.

According to an embodiment of the present application, the step S1 further comprises: passing the heavier hydrocarbon to be preheated into a first heating section 1A for first preheating to obtain a first preheated heavier hydrocarbon; respectively introducing at least a portion of the first preheated heavier hydrocarbon and a heat exchange medium into a heat exchanger 6A for heat exchange to obtain a second preheated heavier hydrocarbon; then passing the second preheated heavier hydrocarbon into the first vapour-liquid separator 8 as the preheated heavier hydrocarbon; wherein the heat exchange medium comprises steam heated by a heater 6B, or steam heated by the second heating section 2, or steam from a steam source.

According to an embodiment of the present application, the step S1 further comprises: introducing at least a portion of the first preheated heavier hydrocarbon into a heater 6B for heating to obtain a third preheated heavier hydrocarbon; and then passing the third preheated heavier hydrocarbon into the first vapour-liquid separator 8 as the preheated heavier hydrocarbon.

According to an embodiment of the present application, the step S1 further comprises: passing at least a portion of the first preheated heavier hydrocarbon from the first heating section 1A into the heavier hydrocarbon preheating section 1B for heating to obtain a fourth preheated heavier hydrocarbon, and passing the fourth preheated heavier hydrocarbon into the first vapour-liquid separator 8 as the preheated heavier hydrocarbon; or, optionally, passing at least a portion of the first preheated heavier hydrocarbon from the first heating section 1A into the heavier hydrocarbon preheating section 1B, and then passing it through the heat exchanger 6A and/or the heater 6B for heat exchange and/or heating, to obtain a fourth preheated heavier hydrocarbon, and passing the fourth preheated heavier hydrocarbon into the first vapour-liquid separator 8 as the preheated heavier hydrocarbon; or, passing at least a portion of the first preheated heavier hydrocarbon from the first heating section 1A into the heavier hydrocarbon preheating section 1B to obtain a fourth preheated heavier hydrocarbon, and passing another part of the first preheated heavier hydrocarbon from the first heating section 1A into the heat exchanger 6A and/or the heater 6B for heat exchange and/or heating, and then mixing the resultant with the fourth preheated heavier hydrocarbon, and passing the mixed heavier hydrocarbon into the first vapour-liquid separator 8 as the preheated heavier hydrocarbon. Specifically, the mode for preheating the heavier hydrocarbon may be selected according to the heat actually required by the steam cracking system.

According to an embodiment of the present application, the heat exchange medium may be any heat exchange medium conventionally used in the art, and is not limited to the steam listed.

According to an embodiment of the present application, the step S1 further comprises: subjecting the first preheated heavier hydrocarbon to desalting, and then to heat exchanging and/or heating, to further heat the first preheated heavier hydrocarbon by using flue vapour obtained by the desalting pretreatment; or subjecting the heavier hydrocarbon to be preheated to desalting, and then passing it into the first heating section 1A for first preheating. By coupling the steam cracker with an electric desalter and adding an external heater and a heat exchanger, the present application is beneficial to the comprehensive utilization of energy, the reduction of energy consumption and the improvement of applicability to heavier hydrocarbons.

According to an embodiment of the present application, in the step S2, at least a portion of the primary dilution steam is passed into the third heating section 3A for heating, to obtain a superheated primary dilution steam, and the superheated primary dilution steam and the first liquid stream are subjected to first mixing, wherein the temperature of the superheated primary dilution steam is 180-400 °C, preferably 200-350 °C.

According to an embodiment of the present application, the second vapour stream comprises entrained steam and a second lighter fraction; the second liquid stream comprises a second heavier fraction. Preferably, the second vapour stream comprises entrained steam and the second lighter fraction; the second liquid stream comprises the second heavier fraction.

According to an embodiment of the present application, the first lighter fraction accounts for 30-40 wt% of the total weight of the heavier hydrocarbon, the first lighter fraction has an initial boiling temperature of less than or equal to 270 °C, and a final boiling temperature of between 265 °C and 275 °C; the first heavier fraction is in phase equilibrium with the first lighter fraction; the second lighter fraction has a final boiling temperature of 330-480 °C; the second heavier fraction is in phase equilibrium with the second lighter fraction; the third lighter fraction mainly comprises C1-C6 components, and has a final boiling temperature of less than or equal to 90 °C, and the third lighter fraction is sent to other cracking furnaces for cracking; the first intermediate fraction mainly comprises C6-C10 components, and has a distillation range of 80-170 °C, and the first intermediate fraction is sent to a reforming unit; the third heavier fraction has a distillation range of 180-380 °C, and mainly comprises components having 11 or more carbon atoms. It can be appreciated that the initial boiling point or the final boiling point of each fraction in the present application is a range value, and any temperature within the range value may be possible in the actual operation, and the fraction to be separated can be selected in the present application according to the actual production needs. The first intermediate fraction of the present application has a high potential aromatic content, and thus is not suitable for steam cracking, can be flexibly sent to oil refining units, such as reforming units and the like, to serve as feedstock, so that it can be used to produce aromatics, olefins and oils wherever appropriate.

According to an embodiment of the present application, the steam cracker comprises a convection zone and a radiant zone from top to bottom. Along the height direction of the steam cracker, the convection zone comprises a first heating section 1A, an optional heavier hydrocarbon preheating section 1B, an optional second heating section 2, a third heating section 3A, a fourth heating section 4, a fifth heating section 3B, and a sixth heating section 5.

According to an embodiment of the present application, the first mixed stream is passed into the fourth heating section 4 for first heating, and then is subjected to the second mixing with a secondary dilution steam superheated by the fifth heating section 3B to obtain a second mixed stream. In addition, the second vapour stream is passed into a sixth heating section 5 for second heating and then is passed into the radiant zone for steam cracking.

According to an embodiment of the present application, the first vapour-liquid separator 8 is configured to subject the preheated heavier hydrocarbon to a first separation therein, to obtain a first vapour stream and a first liquid stream. The radiant zone of the steam cracker is configured to conduct steam cracking on at least a portion of the first liquid stream to obtain a cracked product comprising olefins.

According to an embodiment of the present application, the separation column 7 is configured to receive at least a portion of the first vapour stream for a second separation, to obtain at least a third lighter fraction, a first intermediate fraction and a third heavier fraction. According to the present application, the separation column 7 is a separator conventionally used in the art, and comprises a conventional condenser, a reboiler and other structures, which can be determined according to actual needs.

According to an embodiment of the present application, the system further comprises a post-treatment unit configured to perform a post-treatment on the first intermediate fraction.

According to an embodiment of the present application, the convection zone is provided with a feedstock heating inlet and a feedstock heating outlet, and the radiant zone is provided with a steam cracking inlet and a cracked product outlet.

According to an embodiment of the present application, the first vapour-liquid separator 8 is provided with a vapour-liquid separation inlet, an optional third heavier fraction circulation inlet, an optional cooling aid inlet, a first vapour stream outlet and a first liquid stream outlet. The vapour-liquid separation inlet of the first vapour-liquid separator 8 is communicated with the feedstock heating outlet of the convection zone, and the first liquid stream outlet of the first vapour-liquid separator 8 is communicated with the steam cracking inlet of the radiant zone.

According to an embodiment of the present application, the separation column 7 is provided with a first vapour stream inlet, and with a third lighter fraction outlet, a first intermediate fraction outlet and a third heavier fraction outlet arranged in sequence from top to bottom along the height direction of the separation column 7. The first vapour stream inlet of the separation column 7 is communicated with the first vapour stream outlet of the first vapour-liquid separator 8.

According to an embodiment of the present application, the cooling aid inlet comprises a liquid hydrocarbon inlet and/or a water inlet; optionally, there are a plurality of cooling aid inlets, wherein at least one cooling aid inlet is a water inlet and at least one cooling aid inlet is a liquid hydrocarbon inlet.

According to an embodiment of the present application, the second vapour-liquid separator 9 is provided with a second mixed stream inlet, a second vapour stream outlet and a second liquid stream outlet, an optional third heavier fraction inlet, and an optional separation aid inlet. The separation aid inlet comprises a liquid hydrocarbon inlet and/or a water inlet.

According to an embodiment of the present application, there are a plurality of separation aid inlets, wherein at least one separation aid inlet is a water inlet and at least one separation aid inlet is a liquid hydrocarbon inlet.

According to an embodiment of the present application, the separation aid inlet is located above the second mixed stream inlet in the axial direction of the second vapour-liquid separator 9.

According to an embodiment of the present application, the first liquid stream outlet of the first vapour-liquid separator 8 is communicated with the second mixed stream inlet of the second vapour-liquid separator 9. A primary dilution steam inlet and a superheated secondary dilution steam inlet are provided on a communicating pipeline between the second mixed stream inlet of the second vapour-liquid separator 9 and the first liquid stream outlet of the first vapour-liquid separator 8. Along the flowing direction of the stream, the primary dilution steam inlet is disposed at the upstream of the superheated secondary dilution steam inlet; optionally, the third heavier fraction outlet of the separation column 7 is communicated with the third heavier fraction circulation inlet of the first vapour-liquid separator 8 and the third heavier fraction inlet of the second vapour-liquid separator 9, respectively. The second vapour stream outlet of the second vapour-liquid separator 9 is communicated with the steam cracking inlet of the radiant zone.

The following detailed description of the present application is provided with reference to the accompanying drawings, but the present application is not limited thereto.

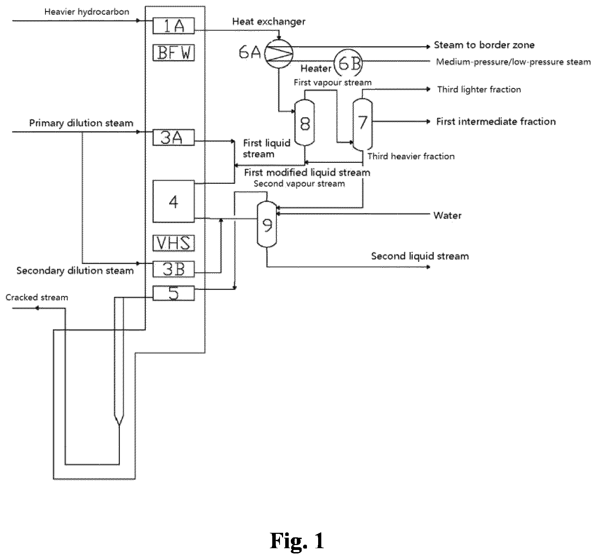

According to an embodiment of the present application, as shown in Fig. 1, the convection zone is provided with a first heating section 1A, an optional boiler feed water preheating section BFW, an optional heavier hydrocarbon preheating section 1B, an optional second heating section 2, a third heating section 3A, a fourth heating section 4, an optional hyperpressure steam superheating section VHS, a fifth heating section 3B, and a sixth heating section 5 from top to bottom, in the height direction of the steam cracker.

The heating inlet of the first heating section 1A is used for introducing heavier hydrocarbon to be preheated; an outlet of the first heating section 1A is communicated with a vapour-liquid separation inlet of a first vapour-liquid separator 8; a pressure regulating valve is provided on an inlet pipeline at the vapour-liquid separation inlet of the first vapour-liquid separator 8 and is used for controlling the preheating temperature of the heavier hydrocarbon entering the first vapour-liquid separator 8; optionally, a pressure regulating valve is provided on the outlet pipeline at the first vapour stream outlet to further control the vaporization rate.

The first liquid stream outlet of the first vapour-liquid separator 8 is communicated with a heating inlet of the fourth heating section 4 through a first pipeline; the heating inlet of the third heating section 3A is communicated with a primary steam source; the heating outlet of the third heating section 3A is connected to the first pipeline.

A heating outlet of the fourth heating section 4 is communicated with the second mixed stream inlet of the second vapour-liquid separator 9 through a second pipeline; the heating inlet of the fifth heating section 3B is communicated with a secondary steam source; the heating outlet of the fifth heating section 3B is connected to the second pipeline.

A heating inlet of the sixth heating section 5 is communicated with a second vapour stream outlet of the second vapour-liquid separator 9; a heating outlet of the sixth heating section 5 is communicated with the steam cracking inlet of the radiant zone.

Optionally, the second liquid stream outlet of the second vapour-liquid separator 9 may be communicated with a hydrocracking or catalytic cracking unit.

Optionally, the second vapour-liquid separator 9 is internally provided with a structure of a tray, a hydrocyclone or a combination of both.

According to an embodiment of the present application, the system further comprises a heavier hydrocarbon preheating unit for further conducting a heat exchange on the first preheated heavier hydrocarbon from the first heating stage 1A to control the temperature of the heavier hydrocarbon entering the first vapour-liquid separator 8.

According to an embodiment of the present application, the heavier hydrocarbon preheating unit comprises a heavier hydrocarbon heating inlet and a heavier hydrocarbon heating outlet. The heavier hydrocarbon heating inlet is communicated with the heating outlet of the first heating section 1A, and the heavier hydrocarbon heating outlet is communicated with the vapour-liquid separation inlet of the first vapour-liquid separator 8.

According to an embodiment of the present application, the heavier hydrocarbon preheating unit comprises a heat exchanger 6A and optionally a heater 6B; the heat exchanger 6A comprises a first heating inlet for heavier hydrocarbon, a heat exchange medium inlet, a first heating outlet for heavier hydrocarbon and an outlet for heat exchange medium after heat exchange; the first heating inlet for heavier hydrocarbon is formed as a heavier hydrocarbon heating inlet of the heavier hydrocarbon preheating unit and is communicated with the heating outlet of the first heating section 1A; a first heating outlet for heavier hydrocarbon, formed as a heavier hydrocarbon heating outlet of the heavier hydrocarbon preheating unit, is communicated with the vapour-liquid separation inlet of the first vapour-liquid separator 8.

According to an embodiment of the present application, the heater 6B comprises a first heating inlet for heat exchange medium and a first heating outlet for heat exchange medium; the second heating section 2 comprises a second heating inlet for heat exchange medium and a second heating outlet for heat exchange medium; the first heating inlet for heat exchange medium and the second heating inlet for heat exchange medium are respectively communicated with a steam source.

According to an embodiment of the present application, the heat exchange medium inlet of the heat exchanger 6A is communicated with the first heating outlet for heat exchange medium of the heater 6B. The heat exchange medium inlet of the heat exchanger 6A is communicated with the second heating outlet for heat exchange medium of the second heating section 2; or the heat exchange medium inlet of the heat exchanger 6A is communicated with a steam source.

According to an embodiment of the present application, the heavier hydrocarbon preheating unit comprises a heater 6B; the heater 6B comprises a second heating inlet for heavier hydrocarbon and a second heating outlet for heavier hydrocarbon; the second heating inlet for heavier hydrocarbon, formed as a heavier hydrocarbon heating inlet of the heavier hydrocarbon preheating unit, is communicated with the heating outlet of the first heating section 1A; the second heating outlet for heavier hydrocarbon, formed as a second heating outlet for heavier hydrocarbon of the heavier hydrocarbon preheating unit, is communicated with the vapour-liquid separation inlet of the first vapour-liquid separator 8.

According to an embodiment of the present application, the heating outlet of the first heating section 1A is communicated with the heating inlet of the heavier hydrocarbon preheating section 1B, and the heating outlet of the heavier hydrocarbon preheating section 1B is communicated with the vapour-liquid separation inlet of the first vapour-liquid separator 8 via a third pipeline; optionally, the heat exchanger 6A or the heater 6B is connected to a third pipeline.

As shown in Fig. 1, in an exemplary embodiment, the system for producing olefins by steam cracking of a heavier hydrocarbon according to the present application comprises:
a steam cracker, a heavier hydrocarbon preheating unit, a separation column 7, a first vapour-liquid separator 8 and a second vapour-liquid separator 9; the heavier hydrocarbon preheating unit comprises a heat exchanger 6A and a heater 6B;
the steam cracker comprises a convection zone and a radiant zone; along the height direction of the steam cracker, the convection zone is provided with a first heating section 1A, a boiler water feed preheating section BFW, a third heating section 3A, a fourth heating section 4, a fifth heating section 3B and a sixth heating section 5 from top to bottom; the radiant zone comprises a steam cracking inlet and a cracked product outlet;
the heat exchanger 6A comprises a first heating inlet for heavier hydrocarbon, a heat exchange medium inlet, a first heating outlet for heavier hydrocarbon and an outlet for heat exchange medium after heat exchange;
the separation column 7 is provided with a first vapour stream inlet, and with a third lighter fraction outlet, a first intermediate fraction outlet and a third heavier fraction outlet, independent from each other, that are arranged in sequence from top to bottom along the height direction of the separation column 7; the first vapour-liquid separator 8 is provided with a vapour-liquid separation inlet, a third heavier fraction circulation inlet, a first vapour stream outlet and a first liquid stream outlet; the second vapour-liquid separator 9 is provided with a second mixed stream inlet, a second vapour stream outlet, a second liquid stream outlet, a third heavier fraction inlet and a separation aid inlet; wherein, the heating inlet of the first heating section 1A is used for introducing heavier hydrocarbon to be preheated; the heating outlet of the first heating section 1A is communicated with the first heating inlet for heavier hydrocarbon of the heat exchanger 6A; the first heating outlet for heavier hydrocarbon of the heat exchanger 6A is communicated with the vapour-liquid separation inlet of the first vapour-liquid separator 8;
a first heating inlet for heat exchange medium of the heater 6B is communicated with a steam source, and a first heating outlet for heat exchange medium of the heater 6B is communicated with the heat exchange medium inlet of the heat exchanger 6A;
the first liquid stream outlet of the first vapour-liquid separator 8 is communicated with a heating inlet of the fourth heating section 4 through a first pipeline; the heating inlet of the third heating section 3A is communicated with a primary steam source; the heating outlet of the third heating section 3A is connected to the first pipeline;
a heating outlet of the fourth heating section 4 is communicated with a second mixed stream inlet of the second vapour-liquid separator 9 through a second pipeline; a heating inlet of the fifth heating section 3B is communicated with a secondary steam source; a heating outlet of the fifth heating section 3B is connected to the second pipeline;
a heating inlet of the sixth heating section 5 is communicated with a second vapour stream outlet of the second vapour-liquid separator 9; a heating outlet of the sixth heating section 5 is communicated with the steam cracking inlet of the radiant zone;
a first vapour stream outlet of the first vapour-liquid separator 8 is communicated with the first vapour stream inlet of the separation column 7, the third lighter fraction outlet of the separation column is connected to another cracking furnace, and the first intermediate fraction outlet is connected to a reforming unit; the third heavier fraction outlet of the separation column 7 is respectively communicated with the third heavier fraction circulation inlet of the first vapour-liquid separator 8 and the third heavier fraction inlet of the second vapour-liquid separator 9; the second liquid stream outlet of the second vapour-liquid separator 9 is connected to a hydrocracking or catalytic cracking unit.

The process for producing olefins by steam cracking of a heavier hydrocarbon using the system shown in Fig. 1 comprises:
S1, passing the heavier hydrocarbon to be preheated into a first heating section 1A for first preheating to obtain a first preheated heavier hydrocarbon; respectively passing the first preheated heavier hydrocarbon from the first heating section 1A and the second steam heated by the heater 6B into the heat exchanger 6A for heat exchange to obtain a second preheated heavier hydrocarbon;
passing the second preheated heavier hydrocarbon into the first vapour-liquid separator 8 as the preheated heavier hydrocarbon for first separation to obtain a first vapour stream and a first liquid stream;
S2, passing a primary diluted steam into the third heating section 3A for heating to obtain a superheated primary diluted steam; mixing at least a portion of the first liquid stream with the superheated primary dilution steam to obtain a first mixed stream; the first vapour stream comprising a first lighter fraction, and the first liquid stream comprising a first heavy component; passing the first vapour stream into the separation column 7 for second separation to obtain a third lighter fraction, an optional first intermediate fraction and a third heavier fraction;
passing the first mixed stream into the fourth heating section 4 for first heating, and then subjecting it to a second mixing with a secondary dilution steam superheated by the fifth heating section 3B to obtain a second mixed stream;
passing the second mixed stream into a second vapour-liquid separator 9 for a third separation to obtain a second vapour stream and a second liquid stream; the second vapour stream comprising entrained steam and a second lighter fraction; the second liquid stream comprising a second heavier fraction;
introducing a portion of the third heavier fraction and/or a cooling aid into the first heavy component via the liquid outlet pipeline at the bottom of the first vapour-liquid separator 8 to control the wall temperature of the interior tubes in the fourth heating section 4;
introducing a portion of the third heavier fraction and the separation aid through the vapour-phase space at the upper part and/or the middle part of the second vapour-liquid separator 9 to contact with the second vapour stream, so that the heavy component carried in the second vapour stream is transferred into the third heavier fraction and the separation aid; and
passing the second vapour stream into the sixth heating section 5 for second heating, and then passing it into the radiant zone of the steam cracker for steam cracking to obtain a cracked product comprising olefins.

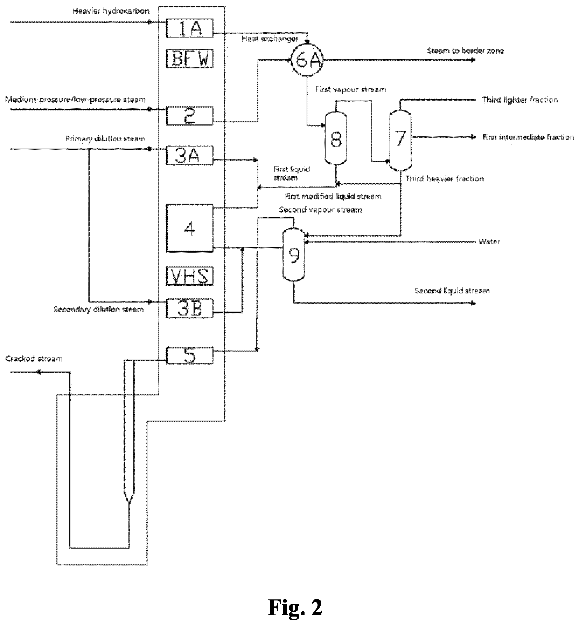

As shown in Fig. 2, in an exemplary embodiment, the system for producing olefins by steam cracking of a heavier hydrocarbon according to the present application is different from the system shown in Fig. 1 in that: the steam cracker is further provided with a second heating section 2 in place of the heater 6B in Fig. 1; a heating outlet of the second heating section 2 is communicated with a heat exchange medium inlet of the heat exchanger 6A; the heat exchange medium is a first steam heated by the second heating section 2.

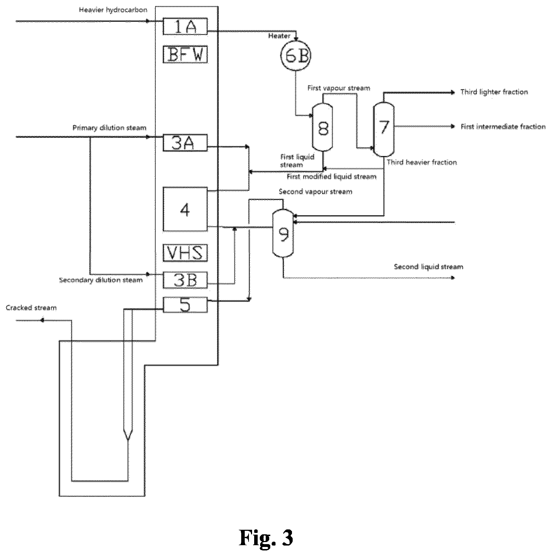

As shown in Fig. 3, in an exemplary embodiment, the system for producing olefins by steam cracking of a heavier hydrocarbon according to the present application is different from the system shown in Fig. 1 in that: the system does not comprise the heat exchanger 6A; the heating outlet of the first heating section 1A is communicated with the heating inlet of the heater 6B, and the heating outlet of the heater 6B is communicated with the vapour-liquid separation inlet of the first vapour-liquid separator 8.

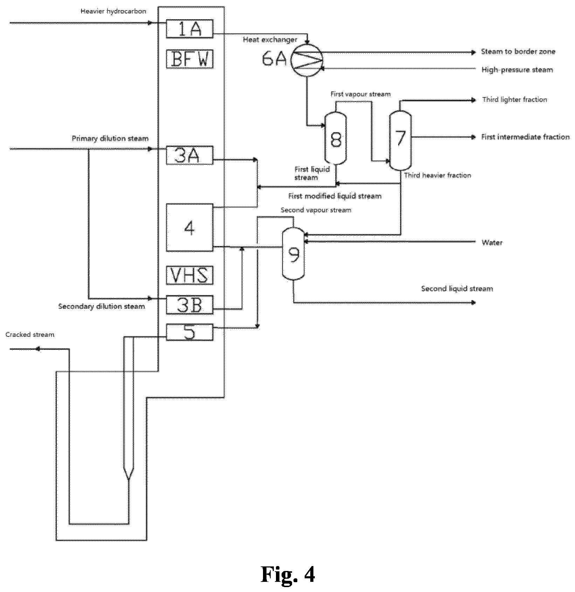

As shown in Fig. 4, in an exemplary embodiment, the system for producing olefins by steam cracking of a heavier hydrocarbon according to the present application is different from the system shown in Fig. 1 in that: the system does not comprise the heater 6B; the heat exchange medium is the third steam; the heating outlet of the first heating section 1A is communicated with the heating inlet of the heater 6B, and the heating outlet of the heater 6B is communicated with the vapour-liquid separation inlet of the first vapour-liquid separator 8.

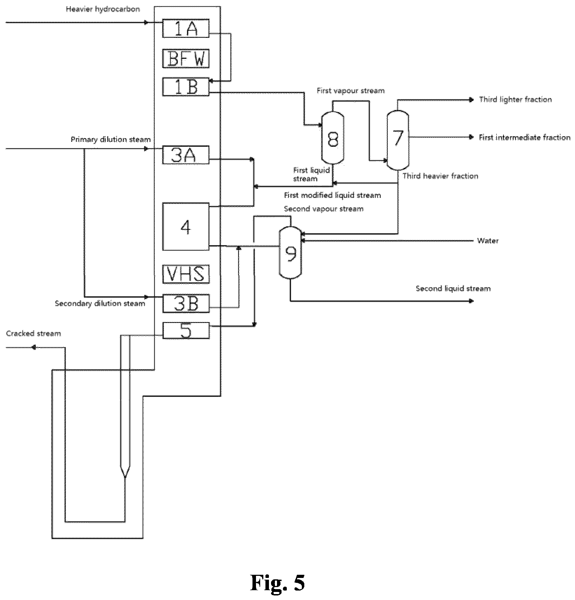

As shown in Fig. 5, in an exemplary embodiment, the system for producing olefins by steam cracking of a heavier hydrocarbon according to the present application is different from the system shown in Fig. 1 in that: the system further comprises a heavier hydrocarbon preheating section 1B, but does not comprise the heat exchanger 6A or the heater 6B; the heating outlet of the first heating section 1A is communicated with a heating inlet of the heavier hydrocarbon preheating section 1B, and a heating outlet of the heavier hydrocarbon preheating section 1B is communicated with the vapour-liquid separation inlet of the first vapour-liquid separator 8.

The embodiment shown in Fig. 5 is the most preferred embodiment of the heat exchange unit of the present application, where the heat source in the steam cracker is incapable of supplying the heat required by the steam cracking reaction or no reboiler is provided at the outlet of the first heating section 1A, a heat exchanger and/or a heater is provided in the steam cracking system, to introduce an external heat source to heat the reaction stream.

The above operating temperatures can be normal operating temperatures in a cracking furnace under conventional operating conditions.

The parameters involved in the steps and the control method, technical principle and the like of the parameters have been described in detail in the foregoing, and thus the detailed description thereof is omitted here for brevity.

### Examples

The present application will be described in further detail below by examples and comparative examples, but the present application is not limited to these examples.

In the following examples and comparative examples, the ethylene yield is calculated as the ratio of the weight of ethylene at the outlet of the radiant zone of the cracking furnace to the weight of heavier hydrocarbon cracked in the radiant zone, and the chemical yield is calculated as the ratio of the total mass of produced chemicals to the total mass of heavier hydrocarbon processed.

In the examples and comparative examples below, the energy consumption in energy efficiency is calculated based on the energy (MW) consumed by the refinery for producing each ton of ethylene.

The energy consumption is calculated according to the index and the calculation method specified in the National Standard for calculation of energy consumption in petrochemical engineering design (GB/T50441-2016).

### Example 1

This example was carried out in accordance with the process flow shown in Fig. 1 using a low-sulfur paraffinic base crude oil having an A PI value of 42.

The distillation range of the heavier hydrocarbon is as follows:

| TBP 760 MMHG (WT) | |
|---|---|
| Distillate mass fraction/% | Temperature/°C |
| Initial boiling point | 17 |
| 10 | 46 |
| 30 | 153 |
| 50 | 248 |
| 70 | 356 |
| 90 | 527 |
| 95 | 625 |
| 98 | 723 |

The heavier hydrocarbon has a content of non-volatile components of 7 wt% and a potential aromatic content of 15 wt%, based on the total weight of the heavier hydrocarbon.

The process for producing olefins by steam cracking of a heavier hydrocarbon of Example 1 specifically comprised the steps of:
S1, preheating heavier hydrocarbon from a storage tank primarily and then subjecting it to a first preheating in a first heating section 1A of a convection zone of a steam cracker, wherein the temperature of the first preheated heavier hydrocarbon was 115 °C; passing the first preheated heavier hydrocarbon into a heat exchanger 6A for further heat exchange with a heat exchange medium to increase the temperature, producing a second preheated heavier hydrocarbon; the heat exchange medium was medium-pressure steam (with a temperature of 350 °C) heated by a heater 6B, and the temperature of the second preheated heavier hydrocarbon was 280 °C;
passing the second preheated heavier hydrocarbon into a first vapour-liquid separator 8 as the preheated heavier hydrocarbon for first separation to obtain a first vapour stream and a first liquid stream;
the first vapour stream had a content of non-volatile components of 0.1 wt% or less, based on the total weight of the first vapour stream;
S2, passing the first vapour stream into a separation column 7 for second separation to obtain a third lighter fraction (with a final boiling temperature of 80 °C), a first intermediate fraction (with an initial boiling temperature of 80 °C and a final boiling temperature of 165 °C) and a third heavier fraction.

### Distillation range of the third heavier fraction

| ASTM D86 | |
|---|---|
| Distillate mass fraction/% | Temperature/°C |
| Initial boiling point | 160 |
| 10 | 190 |
| 30 | 220 |
| 50 | 245 |
| 70 | 287 |
| 90 | 345 |
| 100 | 401 |

mixing the first liquid stream with a primary dilution steam superheated by a third heating section 3A to obtain a first mixed stream; the weight ratio of the first liquid stream to the superheated primary dilution steam was 1: 0.25; the temperature of the superheated primary dilution steam was 340 °C, and the temperature of the first mixed stream was 260 °C;
passing the first mixed stream into a fourth heating section 4 for first heating, wherein the temperature of the first mixed stream after the first heating was 315 °C; subjecting the first mixed stream after the first heating to a second mixing with a secondary dilution steam superheated by a fifth heating section 3B to obtain a second mixed stream; the weight ratio of the first liquid stream to the superheated secondary dilution steam was 1: 0.50; the temperature of the superheated secondary dilution steam was 560 °C;
the temperature of the second mixed stream was 335 °C;
passing the second mixed stream into a second vapour-liquid separator 9 for a third separation to obtain a second vapour stream and a second liquid stream; the second vapour stream comprised entrained steam and a second lighter fraction; the second liquid stream comprised a second heavier fraction (with an initial boiling temperature of more than or equal to 430 °C);
introducing 99 wt% of the third heavier fraction to the first liquid stream such that the weight ratio of the third heavier fraction to the first liquid stream was 17%;
the vaporization rate of the heavier hydrocarbon in the first separation was 35%, and the vaporization rate of the second mixed stream in the third separation was 72%;
passing the second vapour stream into a sixth heating section 5 for second heating and then passing it into a radiant zone of the steam cracker for steam cracking; the steam cracking temperature was 800 °C, and the residence time in the radiant tube was 0.25 s.

In this example, the distillation range of the first vapour stream was:

| TBP at 760MM (WT) | |
|---|---|
| Distillate mass fraction/% | Temperature/°C |
| Initial boiling point | 17 |
| 10 | 35 |
| 30 | 89 |
| 50 | 119 |
| 70 | 164 |
| 90 | 237 |
| 95 | 271 |
| 98 | 321 |

In this example, greater than 95 wt% of the aromatic hydrocarbon material was enriched in the first vapour stream.

The distillation ranges of the first liquid stream and the first modified liquid stream are as follows:

| TBP at 760MM (WT) | First liquid stream | First modified liquid stream |
|---|---|---|
| Distillate mass fraction/% | Temperature/°C | |
| Initial boiling point | 24 | 29 |
| 10 | 111 | 119 |
| 30 | 156 | 162 |
| 50 | 339 | 292 |
| 70 | 440 | 418 |
| 90 | 591 | 564 |
| 95 | 659 | 649 |
| 98 | 723 | 723 |

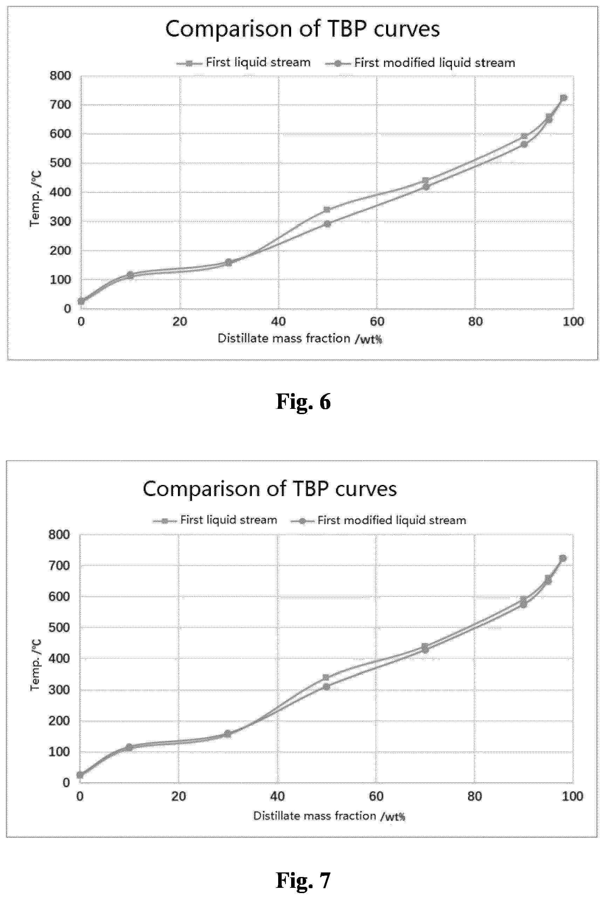

As shown in Fig. 6, by comparing the first liquid stream with the first modified liquid stream, it can be seen that the TBP curve of the first modified material is between 50% and 90% of distillate quantity, the distillation temperature is averagely reduced by about 30 °C, indicating an obvious modifying effect.

According to this example, the 99 wt% of the third heavier fraction was introduced into the first liquid stream, so that the vaporization process of the first modified liquid stream in the fourth heating section was accelerated, no obvious coking phenomenon was observed after a continuous operation for 70 days, the coking of the convection zone can be avoided at a higher operation temperature, and a higher chemical yield can be obtained.

In this example, the temperature of the first mixed stream after the first heating may be controlled at 315 °C, and no significant coking occurred even when the temperature of the first mixed stream was raised to 340 °C.

In this example, the second mixed stream in the third separation was adaptable to a higher temperature by introducing a modifier, which in this example can reach a maximum of 350 °C.

The distillation range of the second vapour stream is as follows:

| TBP at 760MM (WT) | |
|---|---|
| Distillate mass fraction/% | Temperature/°C |
| Initial boiling point | 18 |
| 10 | 143 |
| 30 | 213 |
| 50 | 264 |
| 70 | 300 |
| 90 | 416 |
| 95 | 446 |
| 98 | 501 |
| 100 | 511 |

According to the distillation range of the second vapour stream in this example, the ethylene yield is more than 23 wt% (based on the total amount of crude oil entering the radiant zone for cracking), the triene yield is 42% (based on the total amount of crude oil entering the radiant zone for cracking), and the chemical yield is 71% (the mass ratio of the chemicals generated by cracking the crude oil, excluding PGO, PFO and discharged heavy oil, to the crude oil).

### Example 2

This example was carried out in accordance with the process flow shown in Fig. 2 using a heavier hydrocarbon of the same nature as in Example 1.

The process for producing olefins by steam cracking of a heavier hydrocarbon of this Example specifically comprised the steps of:
S1, preheating heavier hydrocarbon from a storage tank primarily and then subjecting it to a first preheating in a first heating section 1A of a convection zone of a steam cracker, wherein the temperature of the first preheated heavier hydrocarbon was 115 °C; passing the first preheated heavier hydrocarbon into a heat exchanger 6A for further heat exchange with a heat exchange medium to increase the temperature, producing a second preheated heavier hydrocarbon; the heat exchange medium was medium-pressure steam (with a temperature of 350 °C) superheated by a second heating section 2, and the temperature of the second preheated heavier hydrocarbon was 280 °C;
passing the second preheated heavier hydrocarbon into a first vapour-liquid separator 8 as the preheated heavier hydrocarbon for first separation to obtain a first vapour stream and a first liquid stream;
the first vapour stream had a content of non-volatile components of 0.1 wt% or less, based on the total weight of the first vapour stream;
S2, passing the first vapour stream into a separation column 7 for second separation to obtain a third lighter fraction (with a final boiling temperature of 80 °C), a first intermediate fraction (with an initial boiling temperature of 80 °C and a final boiling temperature of 165 °C) and a third heavier fraction, wherein the distillation range of the third heavier fraction is as follows:

| ASTM D86 | |
|---|---|
| Distillate mass fraction/% | Temperature/°C |
| Initial boiling point | 160 |
| 10 | 190 |
| 30 | 220 |
| 50 | 245 |
| 70 | 287 |
| 90 | 345 |
| 100 | 401 |

mixing the first liquid stream with a primary dilution steam superheated by a third heating section 3A to obtain a first mixed stream; the weight ratio of the first liquid stream to the superheated primary dilution steam was 1: 0.25; the temperature of the superheated primary dilution steam was 340 °C, and the temperature of the first mixed stream was 260 °C;
passing the first mixed stream into a fourth heating section 4 for first heating, wherein the temperature of the first mixed stream after the first heating was 315 °C; subjecting the first mixed stream after the first heating to a second mixing with a secondary dilution steam superheated by a fifth heating section 3B to obtain a second mixed stream; the weight ratio of the first liquid stream to the superheated secondary dilution steam was 1: 0.50; the temperature of the superheated secondary dilution steam was 560 °C;
the temperature of the second mixed stream was 330 °C;
passing the second mixed stream into a second vapour-liquid separator 9 for a third separation to obtain a second vapour stream and a second liquid stream; the second vapour stream comprised entrained steam and a second lighter fraction; the second liquid stream comprised a second heavier fraction (with an initial boiling temperature of more than or equal to 430 °C);
introducing 40 wt% of the third heavier fraction into the first liquid stream such that the weight ratio of the third heavier fraction to the first liquid stream was 8%;
the vaporization rate of the heavier hydrocarbon in the first separation was 30%, and the vaporization rate of the second mixed stream in the third separation was 65%;
passing the second vapour stream into a sixth heating section 5 for second heating and then passing it into a radiant zone of the steam cracker for steam cracking; the steam cracking temperature was 805 °C, and the residence time in the radiant tube was 0.25 s.

According to this example, the composition of the third heavier fraction and the first vapour stream was the same as in Example 1, and the amount of the third heavier fraction introduced into the first liquid material was reduced by half as compared with Example 1, so that the modifying effect was limited.

The distillation ranges of the first liquid stream and the first modified liquid stream in Example 2 are as follows:

| TBP at 760MM (WT) | First liquid stream | First modified liquid stream |
|---|---|---|
| Distillate mass fraction/% | Temperature/°C | |
| Initial boiling point | 24 | 27 |
| 10 | 111 | 117 |
| 30 | 156 | 160 |
| 50 | 339 | 311 |
| 70 | 440 | 428 |
| 90 | 591 | 575 |
| 95 | 659 | 651 |
| 98 | 723 | 723 |

As shown in Fig. 7, by comparing the first liquid stream with the first modified liquid stream, it can be seen that the TBP curve of the first modified material is between 50% and 90% of distillate quantity, the distillation temperature is averagely reduced by about 15 °C, the modifying effect is reduced compared with that of Example 1, but is still obtained.

According to this example, 50 wt% of the third heavier fraction was introduced into the first liquid stream, so that the vaporization process of the first modified liquid stream in the fourth heating section was accelerated, no obvious coking phenomenon was observed after a continuous operation for 60 days, the coking of the convection zone can be avoided at a higher operation temperature, and a higher chemical yield can be obtained.

In Example 2, the temperature of the first mixed stream after the first heating may be controlled at 305 °C, and no significant coking occurred even when the temperature of the first mixed stream was raised to 330 °C.

In this example, the second mixed stream in the third separation was adaptable to a higher temperature by introducing a modifier, which in this example can reach a maximum of 340 °C.

The distillation range of the second vapour stream is as follows:

| TBP at 760MM (WT) | |
|---|---|
| Distillate mass fraction/% | Temperature/°C |
| Initial boiling point | 18 |
| 10 | 132 |
| 30 | 219 |
| 50 | 272 |
| 70 | 318 |
| 90 | 418 |
| 95 | 448 |
| 98 | 503 |
| 100 | 511 |

According to this example, the ethylene yield is 22.5 wt% (based on the total amount of crude oil entering the radiant zone for cracking), the triene yield is 40%, and the chemical yield is 69% (the mass ratio of the chemicals generated by cracking the crude oil, excluding PGO, PFO and discharged heavy oil, to the crude oil).

### Example 3

This example was carried out in accordance with the process flow shown in Fig. 4 using a heavier hydrocarbon having an API of 30 and a distillation range shown below:

| TBP 760 MMHG (WT) | |
|---|---|
| Distillate mass fraction/% | Temperature/°C |
| Initial boiling point | 57 |
| 10 | 89 |
| 30 | 241 |
| 50 | 375 |
| 70 | 511 |
| 90 | 595 |
| 95 | 682 |
| 98 | 765 |

The process for producing olefins by steam cracking of a heavier hydrocarbon of Example 3 specifically comprised the steps of:
S1, preheating heavier hydrocarbon from a storage tank primarily and then subjecting it to a first preheating in a first heating section 1A of a convection zone of a steam cracker, wherein the temperature of the first preheated heavier hydrocarbon was 115 °C; passing the first preheated heavier hydrocarbon into a heat exchanger 6A for further heat exchange with a heat exchange medium to increase the temperature, producing a second preheated heavier hydrocarbon; the heat exchange medium was high-pressure steam (with a temperature of 450 °C), and the temperature of the second preheating heavier hydrocarbon was 300 °C;
passing the second preheated heavier hydrocarbon into a first vapour-liquid separator 8 as the preheated heavier hydrocarbon for first separation to obtain a first vapour stream and a first liquid stream; the first vaporization rate of the first separation was 30%;
the first vapour stream had a content of non-volatile components of 0.1 wt% or less, based on the total weight of the first vapour stream;
S2, passing the first vapour stream into a separation column 7 for second separation to obtain a third lighter fraction (with a final boiling temperature of 80 °C), a first intermediate fraction (with an initial boiling temperature of 80 °C and a final boiling temperature of 165 °C) and a third heavier fraction, wherein the distillation range of the third heavier fraction is as follows:

| ASTM D86 | |
|---|---|
| Distillate mass fraction/% | Temperature/°C |
| Initial boiling point | 170 |
| 10 | 201 |
| 30 | 255 |
| 50 | 287 |
| 70 | 325 |
| 90 | 385 |
| 100 | 424 |

mixing the first liquid stream with a primary dilution steam superheated by a third heating section 3A to obtain a first mixed stream; the weight ratio of the first liquid stream to the superheated primary dilution steam was 1: 0.25; the temperature of the superheated primary dilution steam was 340 °C, and the temperature of the first mixed stream was 260 °C;
passing the first mixed stream into a fourth heating section 4 for first heating, wherein the temperature of the first mixed stream after the first heating was 295 °C; subjecting the first mixed stream after the first heating to a second mixing with a secondary dilution steam superheated by a fifth heating section 3B to obtain a second mixed stream; the weight ratio of the first liquid stream to the superheated secondary dilution steam was 1: 0.50; the temperature of the superheated secondary dilution steam was 560 °C;
the temperature of the second mixed stream was 330 °C;
passing the second mixed stream into a second vapour-liquid separator 9 for a third separation to obtain a second vapour stream and a second liquid stream; the second vapour stream comprised entrained steam and a second lighter fraction; the second liquid stream comprised a second heavier fraction (with an initial boiling temperature of more than or equal to 430 °C);
introducing 99 wt% of the third heavier fraction to the first liquid stream such that the weight ratio of the third heavier fraction to the first liquid stream was 12%;
the vaporization rate of the heavier hydrocarbon in the first separation was 30%, and the vaporization rate of the second mixed stream in the third separation was 50%;
passing the second vapour stream into a sixth heating section 5 for second heating and then passing it into a radiant zone of the steam cracker for steam cracking; the steam cracking temperature was 800 °C, and the residence time in the radiant tube was 0.25 s.

According to this example, since the crude oil used had a relatively low API and a relatively high distillation range, both the first vaporization rate and the second vaporization rate were low, and the difference (absolute value) between the first vaporization rate and the second vaporization rate was 20%.

In Example 3, the temperature of the first mixed stream after the first heating may be controlled at 295 °C, and the temperature of the first mixed stream may be raised up to 300 °C.

In this example, the second mixed stream in the third separation was adaptable to a higher temperature by introducing a modifier, which in this example can reach a maximum of 320 °C.

In this example, although the heavier hydrocarbon used was heavier, no obvious coking phenomenon was observed after a continuous operation for 50 days.

In this example, compared with the process of atmospheric and vacuum distillation in combination with steam cracking, the process is shortened, the energy utilization efficiency is improved, and the energy consumption of each ton of ethylene in this example is reduced by 8%.

The distillation range of the second vapour stream is as follows:

| TBP at 760MM (WT) | |
|---|---|
| Distillate mass fraction/% | Temperature/°C |
| Initial boiling point | 67 |
| 10 | 155 |
| 30 | 242 |
| 50 | 299 |
| 70 | 351 |
| 90 | 432 |
| 95 | 445 |
| 98 | 501 |
| 100 | 512 |

According to Example 3, the ethylene yield is 21 wt% (based on the total amount of crude oil entering the radiant zone for cracking), the triene yield is 37%, and the chemical yield is 55% (the mass ratio of the chemicals generated by cracking the crude oil, excluding PGO, PFO and discharged heavy oils, to the crude oil).

### Example 4

This example was carried out in accordance with the process flow shown in Fig. 3 using a heavier hydrocarbon having an API of 30 and a distillation range shown below:

| TBP 760 MMHG (WT) | |
|---|---|
| Distillate mass fraction/% | Temperature/°C |
| Initial boiling point | 57 |
| 10 | 89 |
| 30 | 241 |
| 50 | 375 |
| 70 | 511 |
| 90 | 595 |
| 95 | 682 |
| 98 | 765 |

The process for producing olefins by steam cracking of a heavier hydrocarbon of example 4 specifically comprised the steps of:
S1, preheating heavier hydrocarbon from a storage tank primarily and then subjecting it to a first preheating in a first heating section 1A of a convection zone of a steam cracker, wherein the temperature of the first preheated heavier hydrocarbon was 115 °C; passing the first preheated heavier hydrocarbon into an electric heater 6B for heating to increase the temperature, producing a second preheated heavier hydrocarbon, wherein the temperature of the second preheated heavier hydrocarbon was 400 °C;
passing the second preheated heavier hydrocarbon into a first vapour-liquid separator 8 as the preheated heavier hydrocarbon for first separation to obtain a first vapour stream and a first liquid stream; the first vaporization rate of the first separation was 50%.
Since the first vaporization rate was significantly increased as compared with Example 1/2/3, the load of the separation column 7 was significantly increased.
S5, the first vapour stream had a content of non-volatile components of 0.1 wt% or less, based on the total weight of the first vapour stream;
S2, passing the first vapour stream into the separation column 7 for second separation to obtain a third lighter fraction (with a final boiling temperature of 80 °C), a first intermediate fraction (with an initial boiling temperature of 80 °C and a final boiling temperature of 165 °C) and a third heavier fraction, wherein the distillation range of the third heavier fraction is as follows:

| ASTM D86 | |
|---|---|
| Distillate mass fraction/% | Temperature/°C |
| Initial boiling point | 170 |
| 10 | 266 |
| 30 | 312 |
| 50 | 378 |
| 70 | 495 |
| 90 | 561 |
| 100 | 600 |

The third heavier fraction was significantly heavier than that in Example 1/2/3, and in this example, the modifying effect of the third heavier fraction on the first liquid stream was insignificant.

In this example, no obvious coking phenomenon was observed after a continuous operation for 45 days.

### Example 5

This example was carried out in accordance with the process described in Example 1.

Example 5 further comprised the following steps:
The first intermediate fraction from the third separation was sent to a reformer. The first intermediate fraction reforming feed accounted for about 24 wt% of the total crude oil.

In this example, no obvious coking phenomenon was observed after a continuous operation for 70 days.

### Example 6

This example was carried out in accordance with the process described in Example 1.

Example 6 further comprised the following steps:
subjecting the second mixed stream to the third separation, to obtain a vapour stream (referred to as primary vapour stream) and a liquid stream (referred to as primary liquid stream), and counter-currently contacting the primary vapour stream with 20wt% of the total weight of the third heavier fraction and/or a separation aid (liquid water) to obtain the second vapour stream.

The distillation ranges of the primary vapour stream and the second vapour stream are as follows:

| TBP at 760MM (WT) | Primary vapour stream | Second vapour stream |
|---|---|---|
| Distillate mass fraction/% | Temperature/°C | Temperature/°C |
| Initial boiling point | 20 | 18 |
| 10 | 144 | 143 |
| 30 | 235 | 213 |
| 50 | 277 | 264 |
| 70 | 323 | 300 |
| 90 | 432 | 416 |
| 95 | 469 | 446 |
| 98 | 513 | 501 |
| 100 | 522 | 511 |

It can be seen from Example 6 that by counter-currently contacting the primary vapour stream with 20wt% of the total weight of the third heavier fraction and/or a separation aid (liquid water), the second vapour stream became significantly lighter, and the final boiling temperature can be reduced by about 10 °C with the same second mixed stream and under the same conditions as in Example 1 .

In this example, no obvious coking phenomenon was observed after a continuous operation for 50 days.

In this example, compared with the process of atmospheric and vacuum distillation in combination with steam cracking, the process is shortened, the energy utilization efficiency is improved, and the energy consumption of each ton of ethylene in this example is reduced by 7.6%.

### Example 7

The experiment was carried out as described in Example 1, except that the initial boiling point of the third heavier fraction was 140 °C.

In this example, the ethylene yield is 23.2% and the chemical yield is 71.5%.

In this example, no obvious coking phenomenon was observed after a continuous operation for 50 days.

### Example 8

The experiment was carried out as described in Example 1, except that the initial boiling point of the third heavier fraction was 400 °C.

In this example, the ethylene yield is 20.5% and the chemical yield is 65%.

In this example, no obvious coking phenomenon was observed after a continuous operation for 40 days.

In this example, compared with the process of atmospheric and vacuum distillation in combination with steam cracking, the process is shortened, the energy utilization efficiency is improved, and the energy consumption of each ton of ethylene in this example is reduced by 7%.

### Example 9

The experiment was carried out as described in Example 5, except that the weight ratio of the third heavier fraction in the first vapour stream to the first liquid stream was 4%.

In this example, the first intermediate fraction obtained from the third separation was sent to a reformer. The first intermediate fraction reforming feed accounted for about 18 wt% of the total crude oil, and a portion of the aromatic material was lost compared to Example 5.

According to this example, the distillation range of the third heavier fraction is as follows:

### Distillation range of the third heavier fraction

| ASTM D86 | |
|---|---|
| Distillate mass fraction/% | Temperature/°C |
| Initial boiling point | 140 |
| 10 | 175 |
| 30 | 190 |
| 50 | 209 |
| 70 | 220 |
| 90 | 245 |
| 100 | 300 |

In this example, the final boiling point of the third heavier fraction was relatively low, and thus the distillation range of the second vapour phase was widened, and a portion the aromatic hydrocarbon material was transferred into the second vapour phase. As a result, it was not suitable to use the optimal cracking conditions, and the cracking performance was affected.

In this example, no obvious coking phenomenon was observed after a continuous operation for 60 days.

In this example, the ethylene yield is 22.3 wt% (based on the total amount of crude oil entering the radiant zone for cracking), the triene yield is 39%, and the chemical yield is 68% (the mass ratio of the chemicals generated by cracking the crude oil, excluding PGO, PFO and discharged heavy oils, to the crude oil).

### Comparative Example 1

The experiment was carried out as described in Example 1, except that no separation column 7 for the second separation was provided, and the second vapour stream was fed only to the radiant zone of the steam cracker for steam cracking.

According to this comparative example, the ethylene yield is reduced by 2% compared to Example 1 and the chemical yield is reduced by 20% compared to Example 1. According to this comparative example, after a continuous operation for 35 days, the pressure drop of the convection zone became larger, coking phenomenon was observed, and the energy consumption of the unit was increased by 2% (Kg standard oil/t ethylene) due to the reduction of the ethylene yield.

### Comparative Example 2

The experiment was carried out as described in Example 1, except that no separation column 7 for the second separation was provided, and the first vapour stream was fed to the radiant zone of the steam cracker together with the second vapour stream for steam cracking.

According to this comparative example, the ethylene yield is reduced by 1.5% compared to Example 1, and the chemical yield is reduced by 10% compared to Example 1. According to this comparative example, after a continuous operation for 35 days, the pressure drop of the convection zone became larger, coking phenomenon was observed, and the energy consumption of the unit was increased by 1.5% (Kg standard oil/t ethylene) due to the reduction of the ethylene yield.

### Comparative Example 3

In this comparative example, the heavier hydrocarbon was selected in the same manner as in the examples, except that the heavier hydrocarbon was first separated by an atmospheric and vacuum distillation unit, and then the material suitable for cracking was fed into a cracking furnace and the material suitable for reforming was fed into a reformer for cracking, wherein the steam cracker was operated in accordance with a conventional steam cracking process without a multi-stage flashing equipment. The above combination of the atmospheric and vacuum distillation unit, the conventional steam cracker and the reformer unit was used for the purpose of realizing the same product scheme as in Example 1.

As the process flow for atmospheric and vacuum distillation unit was added in this comparative example, the energy consumption was increased, and compared with Example 1, the energy utilization efficiency was reduced, and the energy consumption of the whole unit was increased by 9.8% (Kg standard oil/t ethylene).

## Claims

1. A process for producing olefins by steam cracking of a heavier hydrocarbon, comprising the steps of:
subjecting the heavier hydrocarbon to a first separation (such as flash) to obtain a first vapour stream and a first liquid stream,
subjecting the first vapour stream to a partial condensation (such as fractional distillation, referred to as the second separation) to obtain a condensate (referred to as the third heavier fraction, preferably having an initial boiling temperature of 165 °C or higher, more preferably having a distillation range of 180-380 °C),
introducing at least a portion (e.g., 50wt% or higher or 80wt% of higher of its total weight) of the third heavier fraction into the first liquid stream to obtain a first modified liquid stream,
subjecting the first modified liquid stream to a third separation (e.g., flash) to obtain a second vapour stream and a second liquid stream, and
subjecting the second vapour stream to a steam cracking to obtain a cracked gas effluent comprising olefins.

2. The process according to claim 1, wherein the final boiling temperature of the heavier hydrocarbon is 540 °C or higher (preferably having an initial boiling temperature of 15 °C and a final boiling temperature of 750 °C or higher), and/or the API index of the heavier hydrocarbon is not less than 32 (preferably 38 or more), and/or the content of non-volatile components of the heavier hydrocarbon is 25 wt% or less (preferably 0.1-5.5 wt%), based on the total weight of the heavier hydrocarbon, and/or the potential aromatic content of the heavier hydrocarbon is 10-40 wt%, based on the total weight of the heavier hydrocarbon.

3. The process according to claim 1, wherein the heavier hydrocarbon is at least one selected from the group consisting of paraffin based crude oil, intermediate based crude oil, naphthenic based crude oil, condensate oil, and refined products.

4. The process according to claim 1, wherein the heavier hydrocarbon is preheated to a temperature of 200-400 °C (preferably 240-300 °C) prior to the first separation.

5. The process according to claim 1, wherein the first vapour stream has a content of non-volatile components of 0.5 wt% or less, based on the total weight of the first vapour stream.

6. The process according to claim 1, wherein the first vapour stream is subjected to the second separation to obtain at least a third lighter fraction (preferably having a final boiling temperature of 90 °C or lower), a first intermediate fraction (preferably having a distillation range of 80-170 °C) and the third heavier fraction.

7. The process according to claim 6, further comprising subjecting the first intermediate fraction to at least one treatment selected from the group consisting of reforming treatment, refinery processing, and isomeric separation.

8. The process according to claim 1, wherein the third heavier fraction has a content of non-volatile components of 1 wt% or less (preferably 0.5 wt% or less), based on the total weight of the third heavier fraction.

9. The process according to claim 1, wherein the weight ratio of the third heavier fraction to the first liquid stream is 5-25% (preferably 15-18%).

10. The process according to claim 1, wherein the first modified liquid stream is subjected to a first mixing with a primary dilution steam to obtain a first mixed stream, then the first mixed stream is subjected to a first heating, then the first mixed stream after the first heating is subjected to a second mixing with a secondary dilution steam to obtain a second mixed stream, and then the second mixed stream is subjected to the third separation to obtain the second vapour stream and the second liquid stream.

11. The process according to claim 10, wherein the temperature of the primary dilution steam is 180-400 °C, preferably 200-350 °C, and/or the temperature of the first mixed stream after the first heating is 400 °C or lower, preferably 250-350 °C, and/or the temperature of the secondary dilution steam is 400-630 °C, preferably 450-600 °C, and/or the weight ratio of the first modified liquid stream to the primary dilution steam is 1: (0.1-0.5), preferably 1: (0.2-0.4), and/or the weight ratio of the first modified liquid stream to the secondary dilution steam is 1: (0.2-0.8), preferably 1: (0.3-0.65), and/or the temperature of the second mixed stream is 280-360 °C (preferably 310-340 °C).

12. The process according to claim 1 or 10, wherein a liquid hydrocarbon is further introduced into the first liquid stream, and/or into the first modified liquid stream, and/or into the first mixed stream, at a weight ratio of the liquid hydrocarbon to the first liquid stream of 1: (0.45-0.85) (preferably 1: (0.5-0.7)).

13. The process according to claim 1, wherein in the first separation, the vaporization rate of the heavier hydrocarbon (referred to as the first vaporization rate) is 15-60%, preferably 25-45%, and in the third separation, the vaporization rate of the first modified liquid stream or the second mixed stream (referred to as the second vaporization rate) is 40-80%, preferably 50-70%.

14. The process according to claim 1, wherein the difference (absolute value) between the first vaporization rate and the second vaporization rate is 15-40% (preferably 20-30%).

15. The process according to claim 1 or 10, wherein the first modified liquid stream or the second mixed stream is subjected to the third separation to obtain a vapour stream (referred to as the primary vapour stream) and a liquid stream (referred to as the primary liquid stream), and at least a portion (such as 50 wt% or less or 20wt% or less of the total weight) of the third heavier fraction and/or a separation aid (also referred to as a cooling aid) is counter-currently contacted with the primary vapour stream to obtain the second vapour stream.

16. The process according to claim 15, wherein the weight ratio of the second vapour stream to the third heavier fraction is 1: (0.01-0.35), preferably 1: (0.05-0.2), and/or the weight ratio of the second vapour stream to the separation aid is 1: (0.01-0.2), preferably 1: (0.05-0.15).

17. The process according to claim 15, wherein the primary vapour stream is firstly counter-currently contacted with the separation aid, and then counter-currently contacted with the third heavier fraction, or the primary vapour stream is firstly counter-currently contacted with the third heavier fraction, and then counter-currently contacted with the separation aid.

18. The process according to claim 15, wherein the separation aid is at least one selected from the group consisting of a liquid hydrocarbon and liquid water.

19. The process according to claim 18, wherein the primary vapour stream is firstly counter-currently contacted with liquid water, and then counter-currently contacted with the liquid hydrocarbon, or the primary vapour stream is firstly counter-currently contacted with the liquid hydrocarbon, and then counter-currently contacted with liquid water, or the primary vapour stream is counter-currently contacted with liquid water and the liquid hydrocarbon at the same time.

20. The process according to claim 12 or 15, wherein the liquid hydrocarbon has a final boiling point of 200-540 °C (preferably 250-450 °C or 300-400 °C), based on the total weight of the liquid hydrocarbon, and is preferably at least one selected from the group consisting of heavy naphtha, aviation kerosene oil and diesel oil .

21. The process according to claim 1, wherein the second vapour stream is subjected to a second heating to a temperature of 500-600 °C (preferably 510-550 °C) prior to the steam cracking, and the content of non-volatile components of the second vapour stream is 1 wt% or less (preferably 0.5 wt% or less), based on the total weight of the second vapour stream.

22. A system for producing olefins by the steam cracking of a heavier hydrocarbon, comprising:
a first vapour-liquid separator (8) configured to perform a first separation (such as flash) of the heavier hydrocarbon, to obtain a first vapour stream and a first liquid stream,
a separator, such as a separation column (7), configured to partially condense (e.g. by fractional distillation, referred to as a second separation) the first vapour stream to obtain a condensate (referred to as a third heavier fraction, preferably having an initial boiling temperature of 165 °C or higher, more preferably having a distillation range of 180-380 °C),
a introduction means configured to introduce at least a portion (such as 50 wt% or higher or 80 wt% or higher of its total weight) of the third heavier fraction into the first liquid stream to obtain a first modified liquid stream,
a second vapour-liquid separator (9) configured to perform a third separation (such as flash) of the first modified liquid stream to obtain a second vapour stream and a second liquid stream,
a steam cracker configured to steam-crack the second vapour stream to obtain a cracked gas effluent comprising olefins.
